# EUROPEAN PATENT APPLICATION

(11) **EP 4 636 541 A1**
(43) Date of publication of application: **22.10.2025**
(21) Application number: 23903252.7
(22) Date of filing: 27.11.2023
(51) Int. Cl.: G06F 3/01, G06Q 50/10, A61B 5/369

(54) **SIGNAL PROCESSING DEVICE, SIGNAL PROCESSING METHOD, AND SIGNAL PROCESSING SYSTEM**

(30) Priority: 16.12.2022 JP 2022201330
(71) Applicant: Sony Semiconductor Solutions Corporation, Atsugi-shi, Kanagawa 243-0014 (JP)
(72) Inventor: OBA, Eiji, Atsugi-shi, Kanagawa 243-0014 (JP)
(74) Representative: Müller Hoffmann & Partner
(86) International application number: PCT/JP2023/042308
(87) International publication number: WO 2024/127958

(57) **Abstract**

The present disclosure relates to a signal processing device, a signal processing method, and a signal processing system capable of preventing BMI control from running away.

A determination unit determines whether or not to permit interaction of a user based on a neural signal corresponding to the intention of the user acquired from the brain of the user via a BMI. The present disclosure is applicable to a gateway device provided between the brain of a user and an external device or network service with which the user interacts in a system implemented using BMI technology.

## Description

### TECHNICAL FIELD

The present disclosure relates to a signal processing device, a signal processing method, and a signal processing system, and more particularly, to a signal processing device, a signal processing method, and a signal processing system enabling the prevention of runaway BMI control.

### BACKGROUND ART

In recent years, research and development of a new interface technology called a brain machine interface (BMI) has been in progress as a mechanism for interfacing with machines.

For example, Patent Document 1 discloses a system that filters data in a neural network environment including the BMI to remove inappropriate content.

### CITATION LIST

### PATENT DOCUMENT

Patent Document 1: Japanese Patent Application Laid-Open No. 2013-8359

### SUMMARY OF THE INVENTION

### PROBLEMS TO BE SOLVED BY THE INVENTION

According to the BMI technology, for example, even a user with disabilities affecting the limbs can control an external device solely with the user's intention, and in the future, such a user may be able to interact with the outside world through a network.

On the other hand, such BMI-based control (BMI control) carries the risk of causing runaway behavior due to the user's consciousness, thoughts, or psychological state.

The present disclosure has been made in view of such circumstances, and it is therefore an object of the present disclosure to enable the prevention of runaway BMI control.

### SOLUTIONS TO PROBLEMS

A signal processing device of the present disclosure includes a determination unit that determines whether or not to permit interaction of a user based on a neural signal corresponding to an intention of the user acquired from a brain of the user via a brain machine interface (BMI).

A signal processing method of the present disclosure includes causing a signal processing device to determine whether or not to permit interaction of a user based on a neural signal corresponding to an intention of the user acquired from a brain of the user via a brain machine interface (BMI).

A signal processing system of the present disclosure includes a gateway device that determines whether or not to permit interaction of a user based on a neural signal corresponding to an intention of the user acquired from a brain of the user via a brain machine interface (BMI).

According to the present disclosure, a determination is made as to whether or not to permit the interaction of the user based on the neural signal corresponding to the intention of the user acquired from the brain of the user via the BMI.

### BRIEF DESCRIPTION OF DRAWINGS

Fig. 1 is a flowchart illustrating a process of synesthesia formation and BMI activation.
Fig. 2 is a flowchart illustrating a reinforcement learning process.
Fig. 3 is a diagram illustrating an example of feedback in the early stages of learning.
Fig. 4 is a block diagram illustrating a configuration example of a gateway device.
Fig. 5 is a flowchart illustrating an operational flow of the gateway device.
Fig. 6 is a diagram illustrating a configuration example of a BMI control system according to an embodiment of the present disclosure.
Fig. 7 is a diagram for describing an example of how to control an external device.
Fig. 8 is a diagram for describing an example of how to use a network service.
Fig. 9 is a flowchart illustrating a flow of monitoring processing performed during the execution of an AI-integrated task.
Fig. 10 is a flowchart illustrating an action-taking process performed by a monitor on the basis of an evaluation result.
Fig. 11 is a flowchart illustrating a flow of information dissemination processing via an avatar.
Fig. 12 is a block diagram illustrating a configuration example of hardware of a computer.

### MODE FOR CARRYING OUT THE INVENTION

Hereinafter, a mode for carrying out the present disclosure (hereinafter referred to as an embodiment) will be described. Note that the description will be given in the following order.

1. BMI technology and its challenges
2. Establishment of neural pathway by BMI
3. Connection to external network by BMI and its challenges
4. Reward mechanism for activating BMI
5. Artificial synesthesia formation and BMI activation
6. Effects produced from use of BMI
7. Overview of technology according to present disclosure
8. BMI control system according to embodiment of present disclosure
   8-1. System configuration
   8-2. Control of external device
   8-3. Use of network service
   8-4. Monitoring performed during execution of AI-integrated task
   8-5. Information dissemination via avatar
9. Hardware configuration example of computer

### <1. BMI technology and its challenges>

Traditionally, a user operates an automobile (vehicle) by perceiving and understanding an environment through his/her eyes, ears, and the like and operating controls such as a steering wheel, an accelerator, and brakes. It is difficult to use a vehicle as a means of transportation without appropriately perceiving an environment and operating controls. Therefore, there are quite a few individuals who cannot use vehicles due to physical functional limitations and have difficulty in traveling.

In this context, automated driving has the potential to become a powerful means of providing essential mobility services for social participation to disabled individuals who used to face significant mobility restrictions. Many disabled individuals have difficulties with accessibility, and their needs are diverse. It is therefore difficult to uniformly determine what type of human machine interface (HMI) should be introduced for use of automated driving.

On the other hand, a new interface technology called a brain machine interface (BMI) has been proposed, the interface technology enabling the user to transmit his/her intention to an automated driving system only by thinking to achieve exchange of various information such as specifying a destination. The BMI can be considered an ultimate interface that enables the user to transmit his/her intention to an automated driving system by means that does not require any physical movement to cause the automated driving system to read a neural signal transmitted from the brain of the user according to the intention and reflect the intention in instruction and control. In other words, in a case where the automated driving system cannot make an appropriate prediction determination, the user gives a necessary complementary instruction to the system by a kind of psychokinesis via the BMI to achieve the movement.

However, a mechanism that is operated in a closed loop where the content of intention transmitted by the BMI is not exposed to the outside world including the third party and the like at all may bring about a new challenge. Therefore, in a case where the BMI is introduced into society as a means of accessibility, a measure to gain social acceptance for the new challenge that may be caused by the interface is required, similar to the introduction of artificial intelligence (AI).

The above challenge is caused by the mechanism using the BMI that is different from the conventional mechanisms based on interaction with the outside world through language and body movements such as gestures, and by which the brain can directly interact with the outside world (without being directly exposed to the third party). Typically, in a case where non-disabled individuals participate in society, they communicate through various means such as speech, text, gestures, and facial expressions in almost all situations. At this time, individuals interact with each other through observable means of communication for causing the third party to evaluate their intention as an action associated with the intention, regardless of whether they are conscious or unconscious. That is, whether they like it or not, in the process of living within society, individuals make decisions and predict, as needed, how their actions may affect the outside world and cause discomfort, injury, joy, or the like by interpreting others' reactions and reflexes, and take action accordingly. Through such interaction with the outside world, normal social life is sustained, and this type of interaction can be considered essential.

If an individual tries to participate in society without being directly perceived by the third party, it is equivalent to affecting the outside world by acting as a kind of invisible person. That is, a mechanism by which one's action and intention are conveyed to the surroundings as visual and auditory information can be considered important in social activities. In the context of interacting with society, in a case where one's action is observed by the third party, or one purchases a product or makes a request, these transactions are performed through tangible procedures, and some records are always left by, for example, issuing receipts or exchanging objects or physical forms. Therefore, individuals naturally acquire and learn behaviors that go against social morals and ethics as boundaries that should not be crossed in the process of living and growing in society. That is, individuals grow while unconsciously learning to act in ways that allow for coexistence within society. Specifically, individuals do not act solely based on their immediate desires, but naturally acquire a form of self-restraint in their decision-making and establish these as behavioral norms for living within society.

However, in a case where a situation where such a restraint behavior does not work at all repeatedly occurs, individuals are unable to make appropriate decisions, which may cause problems in daily life.

A medically known example is congenital insensitivity to pain. A patient who is physiologically incapable of feeling pain at all does not feel pain even when injured, so the development of a neural circuit network (hereinafter, also referred to as a neural network) that triggers preventative responses to avoid injuries does not progress. Even if the patient not feeling pain visually recognizes his/her own injury through bleeding or bone fracture, the development, establishment, and reinforcement of the neural circuit network that make preventative responses/actions to avoid injuries do not progress unless the patient can perceive an injury as "pain" in conjunction with information about the factor causing the injury.

Although the details will be described later, neural circuit networks in humans and other organisms are formed through weak interconnections of numerous immature synapses in the early stages of life. When these synapses interact and influence each other to cause actions to eliminate the causes of perceived pain or cause the visual cortex to capture information about factors that may cause pain, pruning of less involved neural circuits among numerous neural circuits with weak connections generated in the early stages progresses, such as reinforcing neural circuit networks that prompt successful trials to remove pain, even without perceiving it, and neural circuits that lead to injury-preventive measures and the like are reinforced. In practice, before taking preventive actions to avoid injuries, there are actions aimed at achieving something that may lead to injuries, and these actions have both benefits and the risk of injury. There are more complex mechanisms at work, such as neural circuits that transmit signals to suppress neurons desiring benefits, and neural circuits that transmit signals to avoid the risk of injury. It is believed that when success is achieved, that is, when a reward is acquired, dopamine, an excitatory substance in the body, is released for a certain limited period, and this release reinforces and stabilizes synapses along neural circuits that have issued or relayed neural impulses indicating the thought, promoting localized learning.

### <2. Establishment of neural pathway by BMI>

In the BMI technology, neural signals from neurons transmitting "intention" are read by a probe electrode. In practice, it is, however, difficult to "interpret" and decode an abstract intention such as emotions with the probe electrode, that is, directly read a thought itself as an electrical signal via the BMI, and it cannot be achieved even with state-of-the-art technology. Thought information, such as intentions, is materialized at the stages of thought processes in the brain as descriptive data including letters and symbols, and the letters and symbols need to be transmitted through response mechanisms similar to handwriting, keyboard input, a touch panel, and speech input. One of the roles of BMI probe electrodes (neural signal detection probes) is to detect those signals that have been transformed into letters, symbols, speech, or diagrams. Furthermore, another role of BMI probe electrodes is to function as an interface that detects and manipulates response displacements such as forward, backward, left, right, up, down, and push-pull in response to variable (analog) outputs of intention communication to the external environment. The response loop read in the latter is not limited to specific senses. Typically, many of the daily actions and movements of the human body are controlled by using feedback from limited sensory response mechanisms to adjust the amount of movement and the force applied. On the other hand, it is difficult to communicate solely through the above, and people interact by materializing their intentions using verbalized words.

Here, focus on the BMI's function of materializing intentions and thoughts by transforming them into text for keyboard input or speech-equivalent signals, moving away from direct reading of thought contents or analog responses by the BMI.

In a case where an individual transmits his/her intention through typing using a physical keyboard, the individual first verbalize their intentions, then try to input the verbalize intentions using the keyboard according to the spelling of the target language. At that time, in a case where touch typing skills are sufficiently developed, one does not have to consciously think about which finger to move to which key position on the keyboard. Instead, the fingers move according to the spelling of the language in which the intention has been verbalized. In a case where the practice of touch typing is insufficient, keys on the keyboard are visually tracked and are individually operated using an index finger or the like. For the BMI technology to be widely used as an established technology, it is necessary to establish information pathways with communication methods using relatively standardized interfaces, such as letters and symbols.

In a case where intentions are converted into words or characters that can be expressed as text, and a keyboard is used as an information input interface for a device, extended keys are deployed by combining a necessary number of keys with the CAPS key and function keys. In such cases, some people visually follow the keys as they type, while others can type accurately using touch typing. For people like the latter, neural circuits that govern the motor nerves required for finger movements based on the input language have been reinforced through training.

There was a time when means of communication such as pagers reached their peak of popularity. For pagers, it is necessary to input all verbalized information to be transmitted as a katakana string using only a numeric keypad such as a public telephone. Inexperienced users visually checks a table and feedback of display characters of a single-line operation panel, and types while considering the accuracy of input. Among experienced users, there is a user who can naturally perform text input using five fingers of one hand at lighting speed without confirming individual operation inputs. This can be said to be a result of reinforcement and establishment of intention and operation necessary for the fingertip as intention transmission neural circuits of the brain that operates the fingertips. That is, without performing visual feedback correction from the eyes, the neural circuit of the intention to input characters and the fingertip operation command necessary for operating the numeric keypad is subjected to reinforcement learning through repeated use, and the neural circuit network such as the fingertip and the hand is constructed.

Furthermore, Morse code, which has long been used as a means of communication through limited output methods, can be considered as an option. In this case, the intention is transmitted by probing a single signal that can be transmitted on a single-line telephone line with the BMI, instead of by issuing a physical command having different numbers of characters. Which type of neural signal detection probe array to use is determined on the basis of the number of signals of different separable neural circuits that can play the role of the interface function required for reinforcement learning. For example, as the number of neural signal detection probes, one signal such as Morse code or a combination that falls within a range of at most the number of letters in the alphabet is considered appropriate. Since the processing capacity of the human brain is limited, even if the number of electrodes of the neural signal detection probe array is increased to increase the number of simultaneous parallel processes, the brain may reach its processing limit, causing panic, or may self-protectively block information transmission in situations where the brain cannot process information. Therefore, increasing the number of electrodes of the neural signal detection probe array has an adverse effect. In the human body, only the fingers of both hands can be manipulated simultaneously and unconsciously by allocating neural resources through daily life, and what the fingers of both hands can do is limited to playing the piano, touch-typing on a computer keyboard, and the like. It is found that skillfully manipulating all the fingers of a foot is mechanically difficult even when hardware for manipulating all the fingers of the foot is prepared and reinforcement learning is performed.

The BMI as a means of communication has a role of a kind of bag auction that is not directly observed from the outside. Once the communication of intentions is defined, necessary information is passed without interpreting individual detailed movements of hands and fingers in the bag, and reinforcement learning of neural circuits leading to interpretation of the movements of hands and fingers progresses on a side that transmits information and a side that receives information in the process of becoming familiar with transactions.

Here, considering only a simple connection between the brain and the outside world, it is also conceivable to consolidate all information transmission and reception into a single signal, like Morse code. In that case, it is necessary to verbalize intentions and thoughts and further convert the result into temporally modulated signals. This does not cause any problem in an external probe electric circuit. However, when a certain neural pathway continues to be responsible for such conversion within the brain, physiologically localized excessive neural utilization occurs to produce stress, which may lead to self-protective dysfunction. Therefore, rather than focusing on a specific nervous system, it is desirable that learning be performed so that neural signals can be captured by a distributed array of about the number of fingers of one hand. Then, learning may be performed to assign a black-and-white response such as YES/NO, up and down, or left and right to a specific neural signal.

Note that, in a case where intention is transmitted via a highly limited signal output like Morse code, it is also fully possible to read, without directly reading signals of neural circuits in the brain, as performed with BMI, motor commands that control body movements influenced by neural signals, such as those of fingertips, hands, and facial expression muscles, or signals indicating tension actually applied to muscles as a detection target, for example. In this case, the signals can be read in a non-invasive manner.

### <3. Connection to external network by BMI and its challenges>

A system using the BMI technology, which is envisioned in the technology according to the present disclosure, is a mechanism where a user wearing the BMI (that is, a means of communication in which communication with the outside world is completely invisible to the third party) acquires and learns a range of behaviors while balancing between disadvantages that may result from causing harm to others, actions punished due to violation of social norms and laws, or provoking negative reactions from others and disadvantages that the user can personally accept. Then, behavioral decision-making psychology that actively engages in actions leading to increased personal benefits and satisfaction is formed, and the acceptable range of risks is learned and acquired from accumulated experiences.

Originally, non-disabled individuals have a behavioral inhibition ability, taking goal-oriented behaviors without causing undue harm to others, as the primary element that inhibits unlimited behaviors that increase personal satisfaction, as a result of the progress of self-consistent behavioral adjustments within a range where coordination can be achieved, avoiding exposure of the behavioral outcomes to the outside world and becoming subject to social sanctions. Even in a case of wearing the BMI, it is necessary to have such a behavioral inhibition ability from a social perspective.

Although details will be described later, there is no clear connection between the probe electrodes and neurons surrounding the probe electrodes only by inserting and implanting the probe electrodes into a cell group in the brain in an invasive manner, and it cannot be expected to accelerate the development of a neural circuit network between intention-expressing neurons and the probe electrodes. Typically, it takes several years for an infant to keep learning through experiences after birth, and to be able to freely transmit his/her intention with the intention and behavior correctly linked, that is, to form a neural circuit network associated with the behavior. In the acquisition of this experience, among connections between immature neurons unique to infancy, the neurons being temporarily generated in large quantities, connections between neurons that contribute to beneficial experiences are reinforced, and a specific neural circuit network is formed. On the other hand, after infancy, the stabilization of the connections of the specific neural circuits progresses through experience, which makes the generation of a new neural circuit network difficult. Therefore, in order to establish and activate the intention transmission neural circuits of the probe electrodes implanted through invasive surgery, it is necessary to promote the generation of the neural circuit network.

Moreover, an important element that accelerates the generation of an artificial neural circuit network in a self-consistent manner between neurons around the probe electrodes in a part of the brain that governs intention is a reward for an intention transmitter. In considering a mechanism to generate neural circuits using a reward, Pavlov's dog experiment is well known as an example where two events are linked by learning from a psychological and macro perspective. This experiment is an experiment in which the sound of a bell as trigger information is input through the sense of hearing and food is given as a reward, which shows that, as such repeated learning progresses, a signal to produce saliva is transmitted through neural circuits in response to the stimulus of the sound of the bell even if the food is not given. This experiment is conducted through the process in which "the auditory triggers that reinforce neural circuits can be observed and understood by the third party, and furthermore, a reward, such as food, can be visual or olfactory, which can be confirmed by the surrounding third party". The reason why saliva is produced in the reflex process of receiving a reward even if food is not given due to reward learning with food is that the neural circuits connecting the two functions are gradually reinforced and become strongly connected through repeated learning, and the neural circuits are reinforced through a complicated process from a microscopic perspective. It is considered that the reinforcement of the useful neural pathways that yields benefits progresses because specific pathways of the neural circuit network are reinforced through repeated learning that makes the neural connections for acquiring a reward dominant amidst the interplay of excitatory synaptic stimulation and inhibitory stimulation arising from the sense of risk avoidance occurring in the process. As a mechanism to avoid congestion of unimportant information transmission that gives an inhibitory neural signal to the target neural pathway due to firing of weakly connected unnecessary neural pathways, pruning of synapses that create unnecessary neural pathways progresses. However, even if the intention transmission mechanism using the BMI is arranged, through invasive surgery, near a group of neurons that is expected to transmit intentions, there is no means for directly confirming the intention transmission, and the timing of giving a reward as a result cannot be determined. The advantages of adopting the BMI lie in use cases where conventional interfaces such as prosthetic legs, prosthetic arms, or artificial vocal cords cannot be effectively used, that is, the Quality of Life (QoL) is improved by adopting artificial interfaces like the BMI through medical intervention. As a representative example, for patients with advanced stages of amyotrophic lateral sclerosis (ALS ) and the like, the BMI is considered a promising measure because they have many difficulties in using such conventional interfaces.

In a case where the use of eye movements and the like is possible, a configuration where a keyboard is arranged on a monitor to show a combination of character arrays, and intention transmission is performed by sequentially selecting characters using a line of sight is widely used. In the future, with further advancements in medical science, a mechanism where intention transmission is directly read by neural signal detection probes inserted invasively as the BMI, and information is transmitted as electrical signals can be considered. When such a system is established as an interface, it becomes possible to widely exchange information with the outside world over a network, without necessarily requiring the third party to observe and reinterpret the information in the process of intention transmission. At this time, with insufficient feedback from inhibitory functions and excessive development of the neural circuits in the brain necessary for intention transmission, side effects such as acquiring rewards excessively without inhibition may occur in the process of activating the BMI function. There are challenges associated with direct brain-to-external-world connections through the BMI, particularly in terms of being able to transmit intentions without being exposed to the third party.

That is, it opens up the possibility of freely manipulating the internet-connected world as if by psychokinesis, without being detected by the third party.

### <4. Reward mechanism for activating BMI>

In a case where a system using the BMI technology described above is implemented with at least with today's technology, precisely decoding all neural circuits in an individual's brain and accurately detecting every single electrical signal of individual neurons based on intentions is not currently feasible, and it is likely to remain challenging in the future.

When input information exceeds a level of determination in a certain neuron, firing of the neuron occurs, and a firing signal is propagated along an axon. It is known that even if damage occurs in a part of the neural circuit network and propagation along a specific axon is partially inhibited reconstruction of the network to compensate for the damage progresses through recovery rehabilitation. Neural pathways in the brain can constantly and dynamically change due to, for example, a self-healing ability that minimizes disruption to daily life through, for example, early rehabilitation.

Therefore, even when probe electrodes are precisely targeted and set on neurons responsible for transmitting interaction with the outside world through invasive procedures, individual connections of the neural circuit network in the brain are not assigned addresses, so it can be said that connecting probe electrodes to the desired neurons is difficult. Instead, in a case where the region of the cerebral cortex that controls body movements is specified, an arrangement in which neurons in the specified region can exert direct effects is found, and electrodes are implanted and arranged in the specified neurons on a one-to-one basis through invasive procedures, it is possible to capture the firing signals of adjacent neural circuits. However, this alone does not capture signals based on the subject's intentions; therefore, within the neural circuit network, it is necessary to reach a state where the neurons adjacent to the electrodes become excited on the basis of the intentions, and probe electrodes invasively implanted and arranged and capable of detecting firing of neurons adjacent to the probe electrodes detects the firing.

The problem here is how the subject's intention can be coupled to a specific neuron adjacent to a surgically implanted probe electrode. Furthermore, it is important to determine whether the adjacent probe electrodes can guide the subject's intention-transmitting neural signals as more accurate and dense active neural signals, and detect these intention signals that trigger firing with the probe electrode. Even if functional magnetic resonance imaging (fMRI) or other functional response evaluation is performed on the brain in advance, and localized three-dimensional arrangement information of a neural circuit network responsible for intention transmission is specified as a region where neural signals are most easily captured by simulation before surgery, it is difficult to connect an artificially implanted probe electrode to a specific neuron in the same manner as physically connecting known cables in the real world. That is, even if the probe electrode is implanted targeting a certain region, the one-to-one arrangement is extremely difficult and is not realistic as described above. Needless to say, a method can also be considered in which the arrangement of neurons where intention transmission occurs is identified during surgery with the subject remaining conscious by inducing fluorescence in firing of neurons or neurotransmitters released from the ends of surrounding neural networks and identifying a location area using the visualized information as a mark or electrically probing the detected signal, while causing the subject to repeatedly express intentions. However, it takes a long time to perform such a method, and it is time-consuming, burdensome, and fraught with uncertainty.

Therefore, attention is paid to inducing migration and generation of neurons as a mechanism by which human neurons record new knowledge and information. Specifically, rather than precisely arranging the probe electrode array on specific neurons or a neuron group, use a phenomenon where neural circuits are newly formed in a self-consistent manner after the implantation of the electrodes. Although there are still many unknowns about the process of forming neural circuits in the medical field, it is known that in a case where decisions may arise to reach new knowledge or solutions to problems, neural circuit connections to neurons that make the decisions gradually increase.

The following can be achieved by utilizing the development of a new neural circuit network that can acquire rewards. That is, for an activity that may provide detection signals to artificially implanted probe electrodes, by feeding a certain reward back to the brain according to the detection signals, connections to a network of neurons adjacent to the probe electrodes increase in a self-consistent manner, and it is possible to achieve a situation as if the probe electrodes responsible for detecting intentions are directly connected to neurons associated with intention transmission.

However, the formation of the self-consistent network in a self-alignment manner with respect to the probe electrodes does not occur spontaneously, and a mechanism of artificially giving reward feedback to the brain of the subject on the basis of signals amplified and subjected to waveform analysis by an external device via the probe electrodes is required at the same time. If the subject's behavior and an incentive as a reward are visible as in Pavlov's dog experiment, the result of this learning is also easy to observe from the outside. However, it is not desired to observe the behavior itself like the behavior of Pavlov's dogs, and it is difficult to directly observe or control the situation where a new neural network develops on the probe electrodes as the BMI function. The generation of the new neural network around the probe electrodes in a self-consistent manner requires repeated stimulation through reinforcement learning and requires learning of spontaneous behavior (here stimulating neural circuits) called operant conditioning. That is, a process of forming a neural network that causes a stimulus to a certain neural circuit to reach the neurons around the probe electrodes is required.

It cannot be expected that the only signals captured by the electrodes implanted in an invasive manner ensure that the brain reliably senses the reward. Therefore, even with a stimulus from a weak neural circuit that is likely to transmit an intention, it is possible to promote the formation of a neural circuit network responsible for outputting intentions by giving well-responding reward feedback to an existing sense that is already developed such as vision or hearing.

On the other hand, regarding information acquisition from an external device or the like, feedback to the brain through signals using the electrodes inserted in an invasive manner can be considered an advanced application. In particular, for patients with ALS, there are many difficulties, such as the difficulty of obtaining visual information from external devices through free movement of the neck and eyes; therefore, obtaining information and rewards through direct reading of electrical signals from external devices, without relying on existing visual or hearing senses, becomes a viable option. Currently, the most widely used technology as a perceptual BMI is the cochlear implant. For the cochlear implant, by directly applying an electrical signal to an inner ear (cochlea), a pinpoint electrode arrangement on neurons with existing neural pathways can be achieved through implantation. Therefore, the cochlear implant is being introduced as highly effective hearing restoration therapy that does not require reinforcement learning for generating a new neural network for artificially inserted electrodes.

### <5. Formation of artificial synesthesia and activation of BMI>

Since information is extracted through the BMI serving as output of thought-based information transmission, it is necessary to reinforce the connections between the probe electrode array implanted artificially in the brain in an invasive manner and the neural network in some way.

Currently, enabling embedding functionality of BMI is still technically in an experimental stage. Among others, as a promising technology for formation of a probe electrode array implanted through invasive surgery and reinforcing connections of synapses connecting neural circuits connected to actual thinking nerves, activation of the functionality, and network formation, cord blood transplantation, which is one of therapeutic approaches for regeneration of neural networks and is used for treatment of patients suffering from neurological disorders due to damage to neural connections, can be given as an example. Among a myriad of immature synapses connecting neurons immediately after the birth in an infant, many of the connections that have experienced no neurotransmission are gradually eliminated by pruning through experience (of being exposed to rewards through successful experiences or the like), while neural pathways that have experienced signal transmission leading to a reward remain as useful neural pathways. As described above, feedback leading to a reward associated with intention transmission is repeatedly given to a region around the probe electrode array through vision, hearing, taste, and other possible senses via high-density neural synapses even if the region is immature in early stages after implantation, thereby gradually reinforcing the connections. Neurons distributed in a large number of intermediate regions connecting the region of the brain responsible for intention transmission and the implantation site of the probe electrodes that cause the BMI to function are connected by synapses to relay information, which is necessary to activate the BMI function.

As an example of the means of promotion, for example, neural circuits, the formation of newly formed, immature synapses, and the formation and reinforcement of neurons themselves are required. Some attempts at regenerative medicine are also progressing through induced pluripotent stem (iPS) cell transplantation and the like, but in recent years, regeneration of damaged neural circuits in patients suffering from spinal cord injury through cord blood transplantation administration has been reported. Initially, it was confirmed that the neural network leading to beneficial results from the formation of synapses that increased while remaining immature is gradually reinforced. This migration leading to the formation of new connections between neurons has been visualized due to the recent development of live imaging under microscopy, and it is expected that similar neural circuit generation can be achieved even for artificially implanted electrode probes through learning in combination with a mechanism that provides rewards.

By the way, synesthesia is considered a superhuman ability that some people are born with, and it is regarded as a unique sensory experience observed only in a limited number of individuals. Synesthesia, though not yet fully understood, is often described as a sensory experience where well-known instances involve numbers, letters, or shapes appearing in distinct colors in the visual areas.

Synesthesia is believed to be caused by the progress of networking of neural circuits for a specific sensory area of the brain and other sensory areas, the neural circuits being densely generated during infancy to transmit sensory information. By artificially causing the networking of neural circuits in the same manner, neural signals desired to be generated in the thinking area are connected to the detection signals of the artificially implanted electrode probes. In order for the neural signals generated by thought to be transmitted to the nerves adjacent to the probe electrode, and for the probe electrode to accurately capture the firing of those nerves, it is necessary to enable the formation of such a neural circuit network. In the normal process of brain development, beneficial neural networks are activated and retained as memories based on personal experiences, and neural networks that are not useful or effective are considered merely noise in neural transmission, causing delays and confusion in judgment and are known to gradually disappear through a process called "pruning".

The process of activating the BMI requires formation of networks that are reinforced and connected on the basis of specific intention information for a probe electrode array arranged in a matrix that does not exist in natural development, and the process can also be considered forced promotion of formation of neural circuit networks corresponding to artificial synesthesia. Synesthesia that spontaneously manifests in some people is a sensation where number or sounds are perceived as specific colors even though information received from sensory organs is not originally perceived by visual organs as direct colors, the sensation being developed along the progress of reinforcing of the neural network acting on visual areas. Originally, the neural network is eliminated through spontaneous pruning if it does not play the role of acquiring rewards, but in some people, it is believed that synesthesia develops because the neural pathways are reinforced in the development process and retained as a sense of obtaining beneficial rewards. Then, not only the information received by the visual organ and the hearing organ, but even sounds or numbers may gradually and directly affect the visual areas in the sense.

The BMI is similar to an original human neural network in terms of formation and reinforcement of neurons involved in thinking and networks connected to a probe electrode array, and greatly differs from the original human neural network in that networking between neural transmission circuits downstream of the neurons involved in thinking and neural circuits around probe electrodes implanted in an invasive manner, rather than neural circuits of sensory neurons of benefit perception or visual areas of the brain is forcibly promoted. On the other hand, by inducing synesthesia in the visual area while providing immediate rewards each time neural impulses to specific probe electrode arrays are repeatedly successful, it can lead to the sensation that the results are acting on the BMI through synesthesia in some cases. That is, just as sensory information is perceived through synesthesia as another sense, when transmitting intention through the BMI, there is an advantage in obtaining a closed feedback loop within the brain by perceiving the transmitted intention through another perceptual sense, even without visual feedback converted by external devices.

That is, originally, even if there is no color classification for each probe electrode implanted invasively, for example, learning using the identification color displayed on the monitor is repeated according to the arrangement of the detection signal, and artificial synesthesia is learned by reinforcement learning, so that it is possible to obtain information fragments of intention transmission without waiting for signal detection response feedback from an external device, and it is possible to output an intention with less delay (latency) for confirmation. Everyone cannot necessarily acquire this ability through training; therefore, the usability and degree of effectiveness vary from case to case.

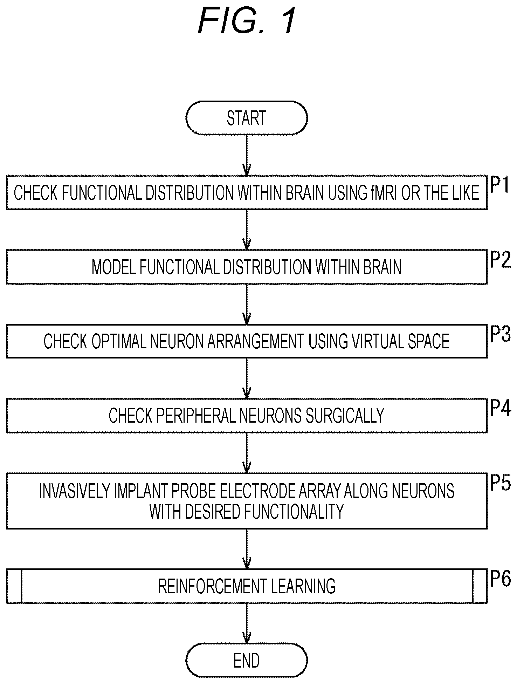

Here, the process of forming artificial synesthesia and activating the BMI described above will be described with reference to the flowchart in Fig. 1.

In a process P1, the functional distribution within the brain is observed using fMRI or the like.

In a process P2, the observed functional distribution within the brain is modeled into a three-dimensional model.

In a process P3, an optimal neural configuration for implanting a probe electrode array is identified on the basis of the modeled three-dimensional model using a virtual space.

In a process P4, neurons around the area where the probe electrode array is implanted are surgically examined in the actual brain of the subject on the basis of the neural configuration identified using the virtual space.

In a process P5, the probe electrode array is invasively implanted along the neurons with the desired function.

In a process P6, reinforcement learning is performed. That is, a new neural network is generated from existing neurons for the probe electrode array implanted in the brain on the basis of the subject's intentions.

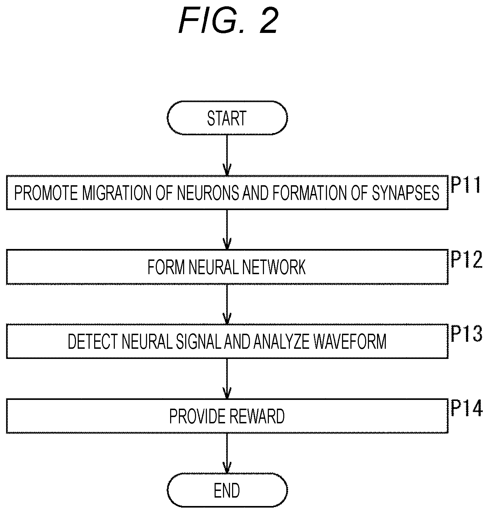

The process of reinforcement learning in the process P6 will be described with reference to Fig. 2.

In a process P11, the migration of neurons and the formation synapses are pharmaceutically promoted through, for example, the administration of cord blood.

This promotes, in a process P12, the generation of the neural network through which neural signals are transmitted to the implanted (embedded) probe electrode array.

Thereafter, in a process P13, a neural signal corresponding to a transmitted intention is detected and its waveforms is analyzed.

In a process P14, in response to the detection of the neural signal (impulse signals) corresponding to the intention from the result of the waveform analysis, a reward for gradually forming and stabilizing the neural network is provided. Here, the "reward" refers to a variety of response results including instruction responses intended by the subject. For example, as a result of the intention to make a selection, if a color can be displayed in response to the intention to display the color, the outcome of the intention appears, providing a form of reward.

Learning is performed by repeating such intention transmission and reward provision. In early stages of learning, rewards are fed back as responses to vision (color, shape, symbol, letter, or the like), hearing, touch, smell, or the like via an external device such as a monitor or a speaker. These are highly transparent information that can be detected by the third party.

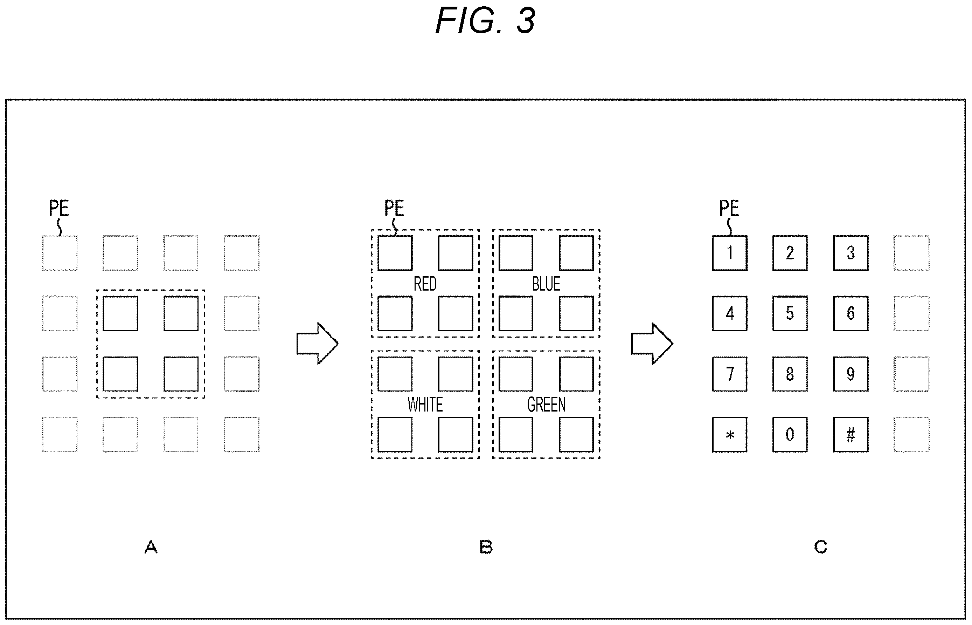

Fig. 3 is a diagram illustrating an example of feedback in early stages of learning.

Fig. 3 illustrates a probe electrode array arranged in a matrix, the probe electrode array including probe electrodes PE arranged in a four by four matrix.

In the initial stage, as illustrated in A of Fig. 3, for example, the reward is provided as a result of detecting a neural signal from any one of the probe electrodes PE of a two by two matrix enclosed by a broken line. The reward is provided in the form of lighting of a lamp, audio output, or the like on the basis of a signal waveform appearing in response to intention transmission. In addition to these, a compliment, a scent, or a perceptual excitatory stimulus to may be provided to the subject.

As the next stage, in a case where a user has normal vision and hearing, as illustrated in B of Fig. 3, the reward is provided as a result of detecting a neural signal obtained by recognizing each of four segments, that is, upper, lower, left, and right segments, of the probe electrode array. For example, a red lamp is caused to light up or blink when a neural signal is detected from any one of the four probe electrodes PE located in the upper left corner, a blue lamp is caused to light up or blink when a neural signal is detected from any one of the four probe electrodes PE located in the upper right corner, a white lamp is cause to light up or blink when a neural signal is detected from any one of the four probe electrodes PE located in the lower left corner, and a green lamp is cause to light up or blink when a neural signal is detected from any one of the four probe electrodes PE located in the lower right corner.

In a subsequent stage, as illustrated in C of Fig. 3, the reward may be provided as a result of detecting a neural signal obtained by recognizing each of the probe electrodes PE of a four by three matrix in the probe electrode array. In the example in C of Fig. 3, numbers from 0 to 9 and symbols * and # are associated on a one-to-one basis with the 12 probe electrodes PE, and a number or symbol corresponding to a probe electrode PE where a neural signal have been detected is displayed on, for example, a monitor.

In this manner, the subdivision of neural circuits progresses, allowing a multiplexed neural signal to be acquired in response to intention transmission.

On the other hand, as learning progresses, signals are directly fed back to neurons via the BMI as responses from the external device. These are non-transparent information that is difficult for the third party to detect.

As described above, after the formation of a neural network for intention output through tangible rewards is established, neural pathways to acquire intangible information (information that is difficult for the third party to detect visually and auditorily) in response to the feedback from the external device are formed. That is, information from the outside is acquired through a new sense.

As described above, by receiving responses from the outside in the form of colors, tactile sensations, and the like different from conventional perceptual information such as characters, artificial synesthesia is formed, and the activation of the BMI progresses. However, the exchange of information that is not read by the third party progresses, and its transparency is lost, which may increase the risk of excessively pursuing rewards and profits.

### <6. Effects produced from use of BMI>

Internet communication has provided a means for dramatically expanding a range of activities as compared with conventional social activities based on human mobility. Then, the spread of today's Internet communication has made it possible to give many opportunities to users who have physical limitations, that is, difficulties with accessibility, to participate in society. Such users suffer from many limitations on not only daily activities such as walking like a non-disabled person, but also speaking, writing, or other forms of normal communication due to illness or the like even with sound brain functions. In such a case, the BMI is an option as an effective interface for interaction with the outside world.

There are even more possibilities of the BMI itself other than allowing movement using, for example, an automated driving system through a means for transmitting intention information using the BMI. As far as automated driving vehicles are concerned, while actual steering itself may be difficult, there is a possibility that roles such as destination instructions and acting as a remote monitor can be sufficiently performed, and it is possible to use an instruction intervention type which is not the automated driving level 4 of Society of Automotive Engineers (SAE). On the other hand, it is not practical to perform invasive surgery for the BMI only for the limited application of automated driving, and only when there are many other additional benefits does the actual implementation become promising. One of the applications is ubiquitous and unlimited exchange of information with the outside world over the Internet or the next generation communication network using the BMI function.

The BMI includes the following characteristics.

1. Generation of the neural network in the brain until the BMI is adequately activated as an interface does not progress without reward feedback. Therefore, a configuration where the feedback information is visible from the outside, that is, visible to the third party is employed. The neural circuit network is constructed through a self-consistent learning process, such as a process in which the reward leads to reinforcement of the neural network.
2. Through a process of verbalizing intentions and converting into letters and symbols, repeated input-output learning advances to response detection equivalent to touch typing.
3. A neural signal detection device of the BMI can be equipped with an error correction function. As learning reaches a certain stage, combining penalties for repeated errors eliminates the need for the process of converting into letters and symbols as an essential element of provided feedback.
4. Dependency on external connection establishment due to relocation or the like is as small as possible, and interconnection can be freely established as needed as long as the relocation destination is within a designated area.
5. Although the function is associated with an individual, it is necessary to restrict the identification of information transmission from the individual due to various privacy constraints such as protection of personal information.
6. Even after the start of use, the BMI and the constructed neural circuit network remain under conditions where continuation, reorganization, and reconstruction may occur on the network with the outside world.
7. In general, the information contains transmitted personal intentions, so it is basically not treated as subject to monitoring and viewing by the third party.
8. The user can freely establish connection to the outside world while staying in a certain private room. Since there are operational limitations as compared to computers or mobile terminals used by general non-disabled individuals, even if all are restricted, it is possible to excel in the ability to translate into a verbalized or symbolized form that can be expressed via the BMI.

On the other hand, since a pathway using the BMI is limited, maximization of the utilization of the pathway leads to maximizing the reward to be acquired, so there is a possibility of runaway behavior occurring in pursuit of rewards.

The use of the BMI enables the establishment of connections with the outside world without using audio or visual text, and as a result, exchange can be performed without the conventional process of materializing information (that is, the exchange of information in the society is exposed to the third party). In early stages of learning for activating the BMI, in the process of formation and development of the neural circuits in the brain, while obtaining visual, auditory, and tactile feedback, synaptic pathways connecting the neural circuits that connect specific intention neurons necessary for information transmission and the probe electrodes constituting the BMI are gradually reinforced (complicated) through experiences of acquiring rewards repeatedly.

Through that process, even without direct feedback such as acquiring rewards, it becomes possible to transmit the desired intentions through the probe electrode array. When such a learning process sufficiently progresses, tangible feedback information such as audio and video, which can be recognized by the third party no longer be necessary for communication. That is, being able to directly act on the probe electrode array with the intention of the brain is equivalent to acquiring the ability to transmit intentions directly with external devices.

In many of the conventional input methods for patients who are unable to move, such as speech, keyboard input, text-based input achieved by a combination of eye gaze and blinks, the information is captured by an intention detection device, converted into display information for confirmation, and displayed on a screen in a format understandable by third parties, thereby allowing the third parties to understand the information. On the other hand, when the BMI technology is established in the future, this series of processes of materializing information becomes unnecessary, and the relationship between users and society undergoes a transformation accordingly.

Furthermore, in a case where various tasks are performed by supplementing a large number of work transactions with external AI processing capabilities, the large number of work transactions are performed only with information transmitted over the network, which is an environment established in a kind of darkness. Unrestricted reward acquisition inherent in the brain with the BMI activated poses a risk of runaway in combination with trial learning for acquiring rewards and maximizing rewards to develop AI functionality, if the suppression mechanism does not work.

In the past, access to information involved physical movement and was through tangible books and magazines. In search operations of electronic terminal devices, provision of unprecedented mobility and accessibility brings about significant changes to thought patterns in various forms such as human information acquisition and memory. However, adaptive learning with AI without being exposed to the third party also leads to opening doors to unknown a certain way. That is, how to suppress the execution of self-serving tasks that may occur as a result of the ongoing process of acquiring rewards, the process being unavoidable for the activation of the BMI, becomes a challenge. In typical social activities, the watchful eyes of society naturally come into play, and when unilateral beneficial actions contradict morals, ethics, or social principles, individuals will learn the risks of sanctions, and self-restraint will naturally be exercised. In addressing this challenge, even if autonomous learning of beneficial tasks is performed through cooperation with external AI via an individual's brain and network, and furthermore, cooperation involving many individuals and AI entities, it is possible to create a situation where learning for acquiring wrong rewards that leads to behavior harmful to others is prevented from progressing by incorporating a mechanism by which the function of monitoring and preventing runaway works continuously, similar to the behavior in general society.

Computers are deeply involved in people's daily lives, and in simulation and various tasks, supplementing thought tasks of individuals with the computers serves as an aspect that undertakes socially beneficial processes. By equipping computers connected to an external network with artificial intelligence, it becomes possible to complementarily utilize such computers according to instructions from the brain, with the brain directly controlling them. By using artificial tools to compensate for physical disabilities, people can exhibit remarkable athletic abilities, as seen in events such as the Paralympic Games. Similarly, in intellectual tasks, cooperation between the brain and AI makes it possible to perform tasks far beyond what an individual's brain can achieve alone. On the other hand, it is difficult to uniformly block and prohibit self-serving reward acquisition through hardware alone when trial tasks to acquire rewards for the activation of BMI progress excessively.

As described above, according to the BMI technology, for example, even a user with physical disabilities in limbs can control an external device solely by the user's intentions, and may also be able to interact with the outside world over networks in the future.

On the other hand, such BMI-based control (BMI control) carries the risk of causing runaway behavior due to the user's consciousness, thoughts, or psychological state.

Therefore, it is necessary to construct a gateway responsible for maintaining social order to ensure the acquisition of benefits or rewards that may result from interaction with the outside world via the BMI and to prevent underground activities while protecting individual privacy and human rights and ensuring a situation that does not lead to individual surveillance or control leading to a surveillance society.

### <7. Overview of technology according to present disclosure>

The technology according to the present disclosure enables the prevention of runaway BMI control, which may be caused by the user's consciousness, thoughts, or psychological state. Note that the technology according to the present disclosure and the effects thereof will be described on the premise of using a BMI requires an invasive surgical procedure to a brain, but can also be applied to an interface function of acquiring a limited number of body surface signals outside the brain as intention signals via probes in a non-invasive manner.

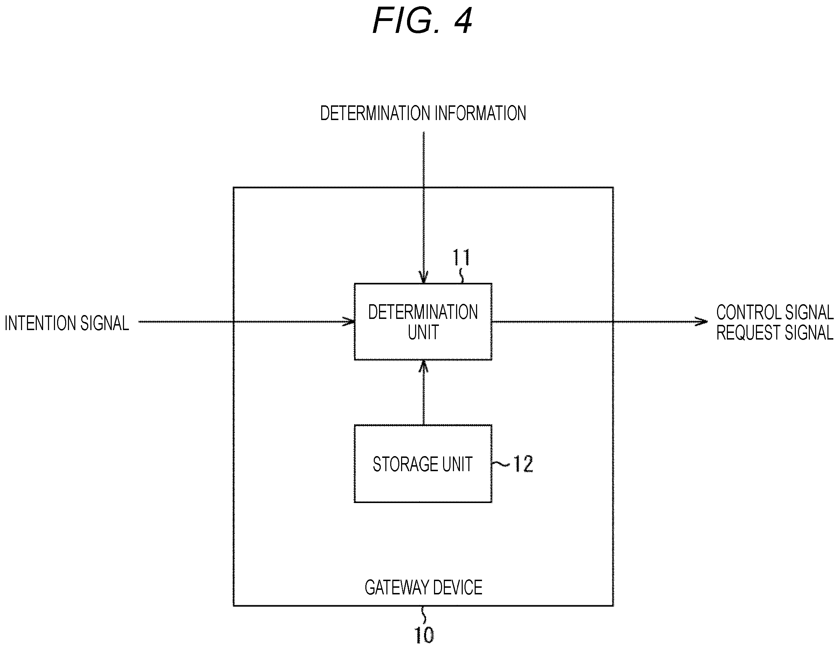

Fig. 4 is a block diagram illustrating a configuration example of a gateway device as a signal processing device to which the technology according to the present disclosure may be applied.

The gateway device 10 illustrated in Fig. 4 may include a general computer such as a personal computer (PC), a mobile terminal such as a smartphone owned by a user, or a server or relay device on a network (cloud) owned by a telecommunications operator, an Internet service provider (ISP), or a service provider providing various network services. The gateway device 10 may be provided between the brain (BMI) of the user and an external device or network service with which the user interacts in a system implemented using the BMI technology.

The gateway device 10 includes a determination unit 11 and a storage unit 12.

The determination unit 11 determines whether or not to permit interaction of the user based on a neural signal corresponding to an intention of the user acquired from the brain of the user via the BMI. Specifically, the determination unit 11 acquires an intention signal obtained by decoding a neural signal acquired from the brain of the user via the BMI, and determines whether or not to permit the interaction of the user based on the intention signal.

The interaction of the user based on the intention signal may correspond to the operation of an external device connected to the BMI in a wired or wireless manner, or may correspond to the use of a network service via a network. In the latter case, the interaction of the user based on the intention signal may correspond to the execution of an AI-integrated task in the network service, or may correspond to information dissemination using an avatar over the network.

The determination unit 11 can also determine whether or not to permit the interaction of the user based on the intention signal using determination information input from the outside or determination information stored in the storage unit 12.

In a case where the interaction of the user corresponds to the operation of an external device, the determination information indicates, for example, the presence or absence of a risk due to a malfunction of the external device. In this case, the determination information may include a consciousness state of the user acquired through monitoring the user, an instruction input based on a third party's judgement, or the like. Furthermore, in a case where the interaction of the user corresponds to the use of a network service, the determination information includes a psychological state or thought content of the user acquired through monitoring the user, a user's confirmation result of feedback on information disseminated by the user, or the like.

The determination unit 11 outputs a control signal to an external device with which the user interacts to operate (control) the external device, or outputs a request signal to a network service with which the user interacts to use the network service, on the basis of the result of the determination as to whether or not to permit the interaction of the user.

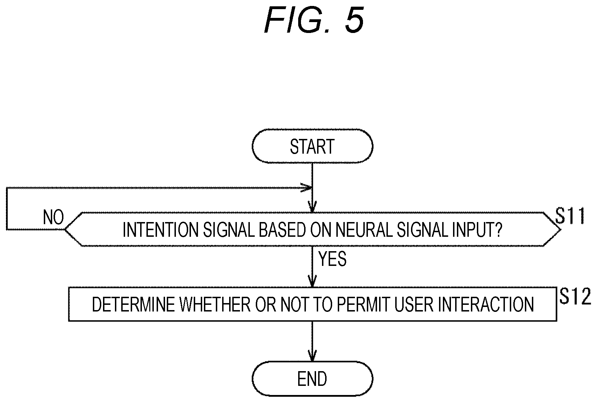

An operational flow of the gateway device 10 will be described with reference to the flowchart in Fig. 5.

In step S11, the determination unit 11 of the gateway device 10 determines whether or not the intention signal based on the neural signal has been input.

Step S11 is repeated until the intention signal is determined to have been input, and when the intention signal is determined to have been input, the processing proceeds to step S12.

In step S12, the determination unit 11 determines whether or not to permit the interaction of the user based on the input intention signal. That is, whether or not to permit the interaction is determined on the basis of whether or not the interaction based on the intention of the user causes harm or disadvantage to the third party. Furthermore, whether or not to permit the interaction based on the intention of the user is determined on the basis of whether or not the interaction is contrary to morals, ethics, or social principles, or there is a risk of excessively pursuing rewards and profits.

The above processing enables the prevention of runaway BMI control, which may be caused by the user's consciousness, thoughts, or psychological state. As a result, it is possible to maintain social order to ensure the acquisition of profits or rewards that may result from interaction with the outside world via the BMI and to prevent underground activities while protecting individual privacy and human rights and ensuring a situation that does not lead to individual surveillance or control leading to a surveillance society.

### <8. BMI control system according to embodiment of present disclosure>

Hereinafter, a configuration and operation example of a BMI control system including the above-described gateway device 10 will be described.

### (8-1. System configuration)

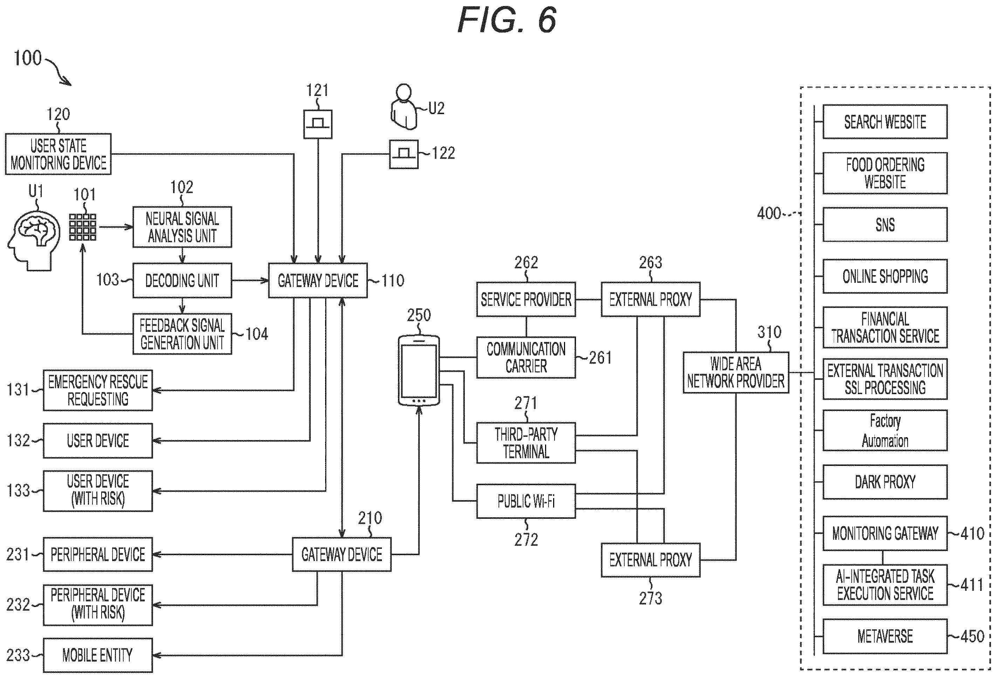

Fig. 6 is a diagram illustrating a configuration example of the BMI control system according to the embodiment of the present disclosure.

The BMI control system 100 illustrated in Fig. 6 includes a probe electrode array 101, a neural signal analysis unit 102, a decoding unit 103, a feedback signal generation unit 104, and a gateway device 110. At least one of the neural signal analysis unit 102, the decoding unit 103, or the feedback signal generation unit 104 may be configured in combination with the gateway device 110.

The probe electrode array 101 is configured as a probe electrode array including probe electrodes arranged in a matrix as described with reference to Fig. 3. The probe electrode array 101 is implanted in the brain of a user U1, and the above-described reinforcement learning is repeated to activate a BMI function of the user U1. A neural signal detected by the probe electrode array 101 is an analog signal including time-series impulse signals and noise. The neural signal detected by the probe electrode array 101 is output to the neural signal analysis unit 102.

The neural signal analysis unit 102 includes a signal processing chip or the like electrically connected to the probe electrode array 101. The neural signal analysis unit 102 removes noise from the neural signal received from the probe electrode array 101, and detects and analyzes a waveform (impulse signals) corresponding to an intention of the user U1. A combination of the analyzed impulse signals (neural signal) is output to the decoding unit 103.

The decoding unit 103 converts the combination of impulse signals (neural signal) received from the neural signal analysis unit 102 into a digital signal, and outputs the digital signal to the gateway device 110 as an intention signal. Furthermore, the intention signal obtained by the digital conversion may be output to the feedback signal generation unit 104 as needed. Note that the decoding unit 103 may include a single signal processing chip including an A/D conversion circuit, or may include a monolithic integrated circuit (IC) integrated with the neural signal analysis unit 102.

The feedback signal generation unit 104 generates a feedback signal for feeding the intention of the user U1 back to the user U1 on the basis of the intention signal received from the decoding unit 103. The feedback signal may be at least one of a display signal for display, an audio signal for audio output, or a BMI signal that input to the brain of the user via the BMI (probe electrode array 101 or another probe electrode array).

Normally, when an individual transmits some intention, for speech, the individual hears his/her own voice and uses feedback to produce proper speech, and for writing or typing, the individual visually checks for errors or inaccuracies and uses feedback to produce accurate text. There is a possibility that errors or the like will occur in the intention output from the brain via the BMI, so some form of feedback loop is required. This feedback loop may be performed through a monitor or speaker perceivable by the third party or through another BMI assigned to an external perceptual input system. Furthermore, feedback can be directly obtained through a cochlear implant already widely in practical use or the like. The function of the feedback loop on the intention transmitted by the user U1 is essential. In a case where there is no feedback loop at all, the brain continuous to be in a situation where it cannot determine whether or not the information transmitted via the BMI is accurate. This is the same situation as when a patient with hearing impairment has difficulty in normal conversations, for example.

The gateway device 110 is an embodiment of the gateway device 10 described with reference to Fig. 4. One or more external devices may be connected to the gateway device 110 in a wired or wireless manner. The gateway device 110 converts the intention signal (neural signal) received from the decoding unit 103 into a control signal for operating (controlling) the external device on the basis of the intention signal, and determines whether or not to permit the input of the control signal to the external device.

A user state monitoring device 120 and control buttons 121 and 122 are connected to the gateway device 110.

The user state monitoring device 120 includes, for example, a computer connected to a camera, a vital sign detection device, and the like, monitors various states of the user U1, and outputs state information indicating the states to the gateway device 110 as the above-described determination information. The states of the user U1 include a consciousness state (consciousness level, whether or not the user is in a sleeping state, whether or not the user is in a coma, and the like), a psychological state (whether or not the user is in an excited state), and thought content (whether or not there is malicious intent).

The control buttons 121 and 122 may each include a mechanical analog button or a UI to be displayed on a touch panel. The control buttons 121 and 122 may be operated by, for example, a third party located near the user U1.

The control button 121 is a button for controlling whether or not to validate the state information output from the user state monitoring device 120. For example, in a case where the state information is validated, the gateway device 110 determines whether or not to permit the input of the control signal to the external device using the state information received from the user state monitoring device 120. Specifically, the gateway device 110 adds the validated state information to the control signal and supplies the resultant control signal to the external device.

The control button 122 is a button for controlling whether or not to enable or disable the input of the control signal from the gateway device 110 to the external device (that is, the BMI function itself). In response to the operation of the control button 122, the gateway device 110 determines whether or not to permit the input of the control signal to the external device on the basis of an instruction input by the third party indicating enabling/disabling of the input of the control signal. For example, in a case where the input of the control signal is disabled, the gateway device 110 does not input the control signal to the external device at all.

The gateway device 110 is connected with an emergency rescue requesting device 131 and user devices 132 and 133 as external devices.

The emergency rescue requesting device 131 includes, for example, a calling device in a nurse call system. The user device 132 is an external device that is worn and carried by the user U1 and located in close proximity to the user U1, and includes, for example, a wearable device, a mobile device, a display device, an audio output device, a bed or chair with an electric reclining function, or the like. The user device 133 is also an external device that is worn and carried by the user U1 and located in close proximity to the user U1, and includes an automated driving wheelchair that is a type of mobile entity, a hydration device for hydration of the user U1, or the like.

Even in a case where the emergency rescue requesting device 131 and the user device 132 malfunction, the risk to the user U1 and their surroundings is minimal, and safety can be maintained. Therefore, these external devices accept the input of the control signal from the gateway device 110, regardless of the state information added to the control signal.

In particular, the user device 132 is familiar to the user such as a monitor or a speaker that transmits information, and is used as a means for confirming a signal produced on the basis of the user's own intention or for a user who cannot speak to communicate his/her intention the third party on the spot. The user device 132 has minimal impact on safety and does not require locking (blocking of the control signal), but may be disconnected, for example, during nighttime sleep, to avoid producing unnecessary information, such as information seen in a dream during sleep.

On the other hand, in a case where the user device 133 malfunctions, there are many risks to the user U1 and their surroundings, which may affect human life and safety. Therefore, these external devices determine whether or not to accept the input of the control signal on the basis of the state information added to the control signal received from the gateway device 110.

In particular, among users who require advanced assistance, there is a patient who needs the help of the third party even for daily hydration and the like, or needs to operate a device under the BMI control. Therefore, depending on the safety and intended use of the external device, a function to block control due to an excessive control time or unusual control, and access control based on the user's abilities and characteristics such as misoperation are required.

In a case where the interaction target of the user having difficulties with accessibility establishes connections with a device always kept close using communication that involves movement around their daily lives, movement to nearby areas, or without movement, the user interacts with a diverse world through the path. Interactions such as control and information acquisition over each connection are switched on the basis of the purpose of the moment. Here, when activating the BMI, a trial is repeated in the formation of thinking nerves, and thoughts that can acquire rewards are retained and learned. As long as such a reward-based learning habit remains instinctive even after establishing the connection to the outside world, and there is no functional degradation that makes the brain lethargic, a reward-based trial is continuously repeated as part of development and evolution. Therefore, especially, with the progress of tasks dynamically integrated with a large number of unspecified external resources, a dynamic blocking mechanism becomes indispensable in order to prevent harmful actions from accelerating in the reward-based trial with the connection established with a dynamically changing outside world.

Therefore, the gateway device 110 is connected with a gateway device 210 connectable to an external network or the like in mainly a wireless manner. The gateway device 110 supplies the intention signal received from the decoding unit 103 to the gateway device 210 together with the state information received from the user state monitoring device 120.

The gateway device 210 is also an embodiment of the gateway device 10 described with reference to Fig. 4. Similarly, one or more external devices may be connected to the gateway device 210 in a wired or wireless manner. The gateway device 210 converts the intention signal received from the gateway device 110 into a control signal, and determines whether or not to permit the input of the control signal to the external device. The state information received from the gateway device 110 is added to the control signal as needed.

Peripheral devices 231 and 232, and a mobile entity 233 are connected to the gateway device 210 as external devices.

The peripheral device 231 is an external device located near the user U1, and includes, for example, an indoor light, a television, a music player, or the like. The peripheral device 232 is also an external device located near the user U1, and includes, for example, an air conditioner such as an indoor air conditioner, an appliance/device involving combustion or heat generation, or the like. The mobile entity 233 includes a vehicle or the like capable of automated driving on the basis of the control signal received from the gateway device 210. The mobile entity 233 may include a wheelchair, a care/living assistance robot, or the like capable of automated driving on the basis of the control signal received from the gateway device 210.

Even in a case where the peripheral device 231 malfunctions, the risk to the user U1 and their surroundings is minimal, and safety can be maintained. Therefore, these external devices accept the input of the control signal received from the gateway device 210, regardless of the state information added to the control signal.

On the other hand, in a case where the peripheral device 232 or the mobile entity 233 malfunctions, there are many risks to the user U1 and their surroundings, which may affect human life and safety. Therefore, these external devices determine whether or not to accept the input of the control signal received from the gateway device 210 on the basis of the state information added to the control signal.

Moreover, a mobile terminal 250 connectable to an external network such as the Internet may be connected to the gateway device 210 in a wireless manner. The mobile terminal 250 includes a mobile phone such as a smartphone owned by the user U1. The user U1 can use network services as interactions via the gateway device 110, the gateway device 210, and the mobile terminal 250.

The mobile terminal 250 is connected to a wide area network provider device 310 via a communication carrier (base station) 261 owned by a telecommunications operator, a service provider 262, and an external proxy server 263.

Furthermore, the mobile terminal 250 may be connected to the wide area network provider device 310 via a third-party terminal 271 owned by the third party and the external proxy server 263, or may be connected to the wide area network provider device 310 via a public Wi-Fi (registered trademark) 272 and the external proxy server 263.

Moreover, the mobile terminal 250 may be connected to the wide area network provider device 310 via the third-party terminal 271 or the public Wi-Fi (registered trademark) 272, and an external proxy server 273 different from the external proxy server 263.

The wide area network provider device 310 is configured as a relay device that connects the mobile terminal 250, that is, the brain of the user U1, and a wide area network or a virtual private network (VPN).

The wide area network provider device 310 is also an embodiment of the gateway device 10 described with reference to Fig 4. A network service 400 that provides various services may be connected to the wide area network provider device 310. The mobile terminal 250 supplies the intention signal received from the gateway device 210 to the wide area network provider device 310, and the wide area network provider device 310 determines the necessity of restrictions on the use of the network service is necessary on the basis of the intention signal received from the mobile terminal 250.

The wide area network provider device 310 has a function to monitor abnormal traffic between the wide area network provider device 310 and the network service 400, anonymous transactions, deliberate excessive tunneling of information communication, and complementary execution processing using external AI, a function to visualize or report such monitoring, and the like.

The network service 400 includes various services provided over the Internet. For example, the network service 400 includes search websites, food ordering websites, social networking services (SNSs), online shopping, financial transaction services, and the like. Furthermore, the network service 400 may include a service for providing secure sockets layer (SSL) processing to prevent tampering or impersonation in external transactions, and a factory automation (FA) service capable of controlling and monitoring a system for automating production processes in a factory.

Furthermore, the network service 400 includes a dark proxy or the like connectable to malicious websites such as dark websites, or includes an AI-integrated task execution service 411 that can execute an AI-integrated task uniquely designed for the BMI via a monitoring gateway 410. In particular, the monitoring gateway 410 has a function to continuously monitor malicious intentional information manipulation in the AI-integrated task, market manipulation in foreign exchange and the like, false information generation, and aiding and abetting prohibited transactions. Furthermore, the monitoring gateway 410 also has functions similar to those of the gateway device 10 described with reference to Fig. 4.

Moreover, the network service 400 may include a metaverse 450 that forms a virtual space on the Internet. In the metaverse 450, the user U1 can perform interaction, such as information dissemination using an avatar, with other remote users via the BMI.

The BMI control system 100 may include components other than the above-described components. Hereinafter, an example of the BMI control in the BMI control system 100 will be described.

### (8-2. Control of external device)

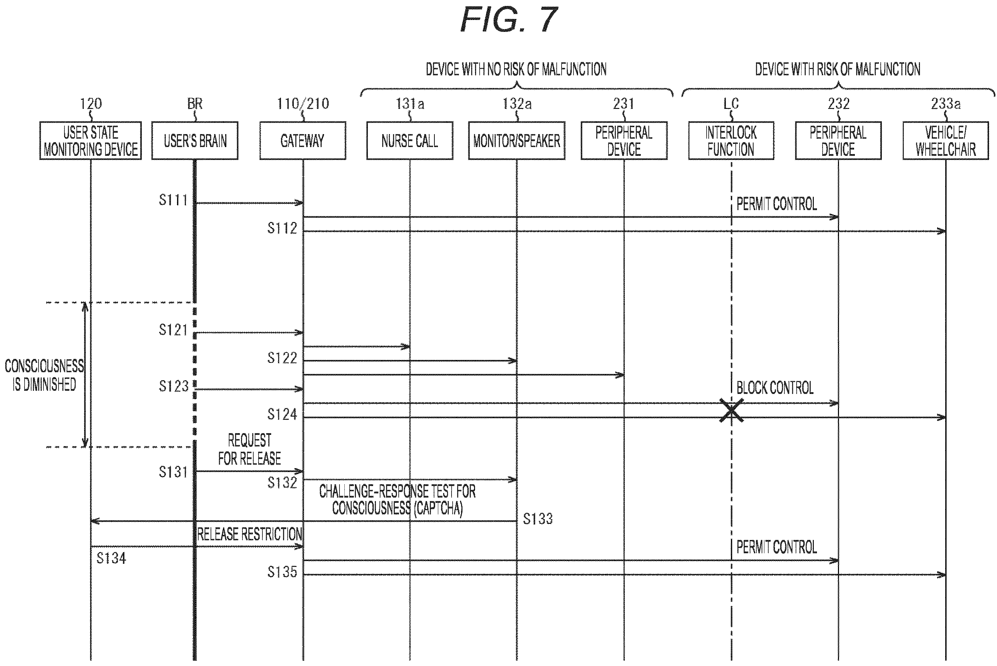

Fig. 7 is a diagram illustrating an example of how to control an external device in the BMI control system 100.

Fig. 7 illustrates an example where the gateway device 110 or the gateway device 210 determines whether or not to permit the input, to the external device, of a control signal converted from a neural signal corresponding to an intention of the user acquired from the brain BR of the user U1 via the BMI. The external device includes a device with no risk of malfunction and a device with the risk of malfunction. The device with no risk of malfunction include a nurse call system 131a as the emergency rescue requesting device 131, a monitor/speaker 132a as the user device 132, and the peripheral device 231. The devices with the risk of malfunction include the peripheral device 231 and a vehicle/wheelchair 233a as the mobile entity 233. The device with the risk of malfunction have an interlock function LC to block the control signal on the basis of the state information added to the control signal.

For example, in step S111, when a neural signal corresponding to an intention to operate the peripheral device 232 or the vehicle/wheelchair 233a is acquired from the brain BR of the user U1 via the BMI, an intention signal converted from the neural signal is supplied to the gateway device 210. At this time, state information indicating that the user is in a normal state of consciousness, which is output by the user state monitoring device 120, is supplied to the gateway device 210 together with the intention signal. Hereinafter, it is assumed that the state information is verified through the control button 121.

In step S112, the gateway device 210 converts the intention signal into a control signal, and determines whether or not to permit the input of the control signal with the state information to the external device. Here, since the state information indicates that the user is in a normal state of consciousness, the input of the control signal to the external device is determined to be permitted, and the peripheral device 231 or the vehicle/wheelchair 233a (interlock function LC) permits (accepts) the input of the control signal.

In step S121, when a neural signal corresponding to an intention to operate the nurse call system 131a, the monitor/speaker 132a, or the peripheral device 231 is acquired from the brain BR of the user U1 via the BMI, an intention signal converted from the neural signal is supplied to the gateway device 110. At this time, the user's consciousness is diminished, for example, the user's consciousness level is low, the user is sleeping, or the user is in a coma, and state information indicating that the user's consciousness is diminished, which is output by the user state monitoring device 120, is supplied to the gateway device 210 together with the intention signal.

In step S122, the gateway device 110 converts the intention signal into a control signal, and determines whether or not to permit the input of the control signal with the state information to the external device. Here, the input of the control signal to the external device is permitted regardless of the state information, and the nurse call system 131a, the monitor/speaker 132a, or the peripheral device 231 accepts the input of the control signal.

Next, in step S123, when a neural signal corresponding to an intention to operate the peripheral device 231 or the vehicle/wheelchair 233a is acquired from the brain BR of the user U1 via the BMI, an intention signal converted from the neural signal is supplied to the gateway device 210. At this time as well, the user's consciousness is diminished, and state information indicating that the user's consciousness is diminished, which is output by the user state monitoring device 120, is supplied to the gateway device 210 together with the intention signal.

In step S124, the gateway device 210 converts the intention signal into a control signal, and determines whether or not to permit the input of the control signal with the state information to the external device. Here, since the state information indicates that the user's consciousness is diminished, the input of the control signal to the external device is determined to be denied, and the peripheral device 231 or the vehicle/wheelchair 233a (interlock function LC) blocks (does not accept) the control signal.

This can reduce the risk to the user U1 and their surroundings due to a malfunction of the peripheral device 231 or the vehicle/wheelchair 233a, and to reduce the impact on human life and safety.

Thereafter, in a case where the user returns to a normal state of consciousness, in step S131, when a neural signal corresponding to an intention to request the release of the state (restricted state) where the control of the external device is restricted is acquired from the brain BR of the user U1 via the BMI, an intention signal converted from the neural signal is supplied to the gateway device 110.

In step S132, upon receipt of the intention signal, the gateway device 110 controls the monitor/speaker 132a to present a challenge-response test to check for the consciousness of the user U1 (challenge-response test for consciousness). Then, in step S133, the monitor/speaker 132a presents, for example, CAPTCHA as the challenge-response test for consciousness of the user U1 to the user U1.

Thereafter, in step S134, the user state monitoring device 120 normalizes the state information and supplies a restriction release signal for releasing the restricted state to the gateway device 210 via the gateway device 110 upon receipt of a thoughtful response made while the user U1 is conscious as the state of the user U1 when the challenge-response test for consciousness is presented.

In step S135, the gateway device 210 determines that the input of the control signal to the external device is permitted on the basis of the restriction release signal, and the peripheral device 231 or the vehicle/wheelchair 233a (interlock function LC) permits (accepts) the input of the control signal with the normalized state information. Note that, even for an external device that affects human life and safety, instead of going through the procedure to check the consciousness of the user U1 for all the control signals, the input of some control signals specified in advance, such as an emergency stop signal, to the external device may be permitted by a simple mechanism with fewer barriers, such as two-step commands, in order to prevent accidents and the like caused by operation delays due to the procedure.

As described above, in the BMI control system 100, it is possible to control an external device while preventing runaway BMI control.

### (8-3. Use of network service)

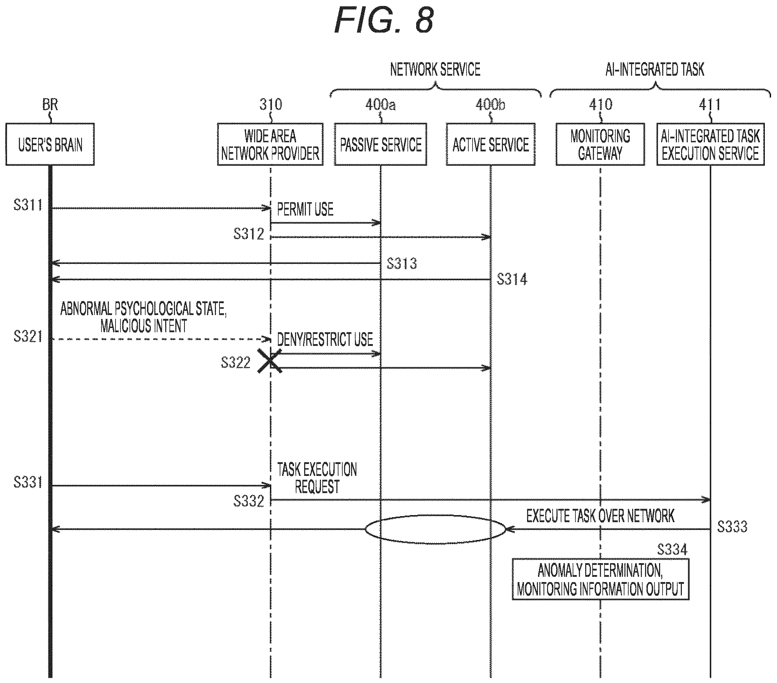

Fig. 8 is a diagram for describing an example of how to use a network service in the BMI control system 100.

Fig. 8 illustrates an example where the wide area network provider device 310 determines the necessity of restrictions on the use of a network service or the presence or absence of an anomaly in an AI-integrated task that is executed in the network service on the basis of the neural signal corresponding to the intention of the user acquired from the brain BR of the user U1 via the BMI. The network service includes a passive service 400a and an active service 400b. The passive service 400a includes services where the user passively acquires information through a procedure, such as searching and browsing. The active service 400b includes services where the user actively exchanges information, such as payment procedures, financial transactions, and online shopping. The AI-integrated task is implemented by the monitoring gateway 410 and the AI-integrated task execution service 411 described above.

For example, in step S311, when a neural signal corresponding to an intention to use the passive service 400a or the active service 400b is acquired from the brain BR of the user U1 via the BMI, an intention signal converted from the neural signal is supplied to the wide area network provider device 310.

In step S312, the wide area network provider device 310 determines the necessity of restrictions on the use of the network service on the basis of the intention signal. Here, the restrictions on the use of the network service are determined to be unnecessary on the basis of a result of determining that the psychological state and thought content of the user U1 are normal, and the use of the passive service 400a and the active service 400b based on a request signal converted from the intention signal is permitted.

As a result, in steps S313 and S314, responses from the passive service 400a and the active service 400b are returned to the brain BR of the user U1.

On the other hand, in step S321, when a neural signal corresponding to an intention to use the passive service 400a or the active service 400b is acquired from the brain BR of the user U1 via the BMI in a state where the psychological state is abnormal or the thought content includes malicious intent, an intention signal converted from the neural signal is supplied to the wide area network provider device 310.

In step S322, the wide area network provider device 310 determines the necessity of restrictions on the use of the network service on the basis of the intention signal. Here, since the psychological state of the user U1 is abnormal or the thought content includes malicious intent, the restrictions on the use of the network service are determined to be necessary, and the use of the passive service 400a and the active service 400b based on a request signal converted from the intention signal is denied or restricted. For example, the use of the passive service 400a and the active service 400b is physically blocked, or viewing, transactions, and the like are restricted. Note that, in a case where risky transactions newly emerge as the use of BMI becomes widespread in society, such as attempts to make inappropriate access to age-restricted information, inappropriate instructions for drug transactions, instructions for information falsification, dissemination of false information, embedding information that causes social unrest, unauthorized access, impersonation transactions, aiding and abetting fraudulent information manipulation, and the like, it is necessary to perform detection, use blocking, or use restriction tailored to such transactions.

As described above, it is possible to monitor the use of the network service by a user who may have an abnormal psychological state or malicious intent.

For example, in step S331, when a neural signal corresponding to an intention to use the AI-integrated task execution service 411 is acquired from the brain BR of the user U1 via the BMI, a control signal converted from the neural signal is supplied to the wide area network provider device 310. The AI-integrated task execution service 411 includes an AI-assisted complementary support service, a self-developing and evolutionary service, and the like, which are uniquely designed for the BMI.

In step S332, the wide area network provider device 310 requests the execution of the AI-integrated task on the basis of the intention signal. Specifically, the wide area network provider device 310 supplies the intention signal to the monitoring gateway 410. The monitoring gateway 410 converts the intention signal received from the wide area network provider device 310 into a request signal for requesting the execution of the AI-integrated task, and supplies the request signal to the AI-integrated task execution service 411.

In step S333, the AI-integrated task execution service 411 executes a user-execution task on the network (network service) with the support of AI.

At this time, the monitoring gateway 410 determines the presence or absence of an anomaly in a task executed in the monitoring processing during the execution of the AI-integrated task, and, in a case where an anomaly in the task is detected, outputs an alert as network service monitoring information (step S334).

### (8-4. Monitoring during execution of AI-integrated task)

In the future, for the use of BMI-based network services, it is anticipated that an interface capable of directly connecting to various services such as metaverse to allow information transmission from a service provider company without relay conversion will be provided. On the other hand, for the time being, various services provided over the regular Internet are based on interfaces designed for non-disabled users.

At this time, in a case where various services disclosed on the network with communication contents equivalent to those for non-disabled individuals are used by a mobile terminal owned by an individual wearing the BMI, it is not necessary to perform processing such as monitoring and anomaly detection. However, in the future, as AI-integrated tasks continue to exceed human abilities, not only beneficial applications, such as converting information representing an intention, transmitted via the BMI, of a user who has lost the ability to speak due to illness into natural speech emerge, but also risks that external AI resources are misused for complementing arise, evolving to the point where information can be manipulated at will. It is therefore necessary to effectively detect the occurrence of the following: execution of inappropriate tasks or integrated tasks; generation of tasks that excessively acquire rewards; tunneling of transmission tasks, excessive concealment and evasion of monitoring; and tasks that evolve flexibly and are executed in response to countermeasures against laundering operations and the like, to identify those that may lead to antisocial information manipulation.

As described above, the BMI enables the creation of new possibilities for human abilities. On the other hand, leveraging the processing power of external artificial computers for operations that used to require human input through speech or keyboard operations enables processing far exceeding human abilities to be performed solely by intention. In a case where such processing becomes the norm, the society may be divided between BMI users who perform the processing and non-disabled individuals who live without BMI, resulting in negative effects such as excessive competition in a coexisting society.

The above impact leads to excessive competition in various aspects, such as profit-making and ticket purchases influenced by minimal time differences, and transactions in volatile markets, resulting in a loss of rational humanity; therefore, it becomes necessary to implement a regulating function to regulate artificial transactions. Even if transactions that gain legal rewards are performed by executing an AI-integrated task that complements human abilities in accordance with instruction information based on human intention transmitted via the BMI, achieving excessively high productivity and rewards by an ordinary individual using the AI-integrated task without the intervention of the BMI leads to unnecessary competition in social activities, resulting in a situation that risks a loss of humanity. Therefore, the specifics of the regulating function will need to be reviewed and updated as needed, as the BMI becomes more widespread in society in the future.

On the other hand, in the technology according to the present disclosure, from an ethical standpoint in society, filtering of artificially introduced processing and assignment of a task transaction necessity and permission flag are performed.

For example, it is considered to implement the following functions.

- Establishment of a communication protocol specialized for information transmission by BMI and dynamic instruction of external AI, assignment of a permission flag to profit-seeking processing, assignment of an ethical permission flag, assignment of a detection flag to automated replication and proliferation processing, and assignment of a verification flag for execution owner information
- Management of linkage of command systems using blockchain technology, in order to prevent falsification of user information at the source of commands when executing malicious tasks using external AI services
- Detection of abnormal communications and abnormal procedures from traffic monitoring, and notification to administrator
- Detection of excessive concealment processing on communication content and unauthorized information concealment, and notification to administrator
- Monitoring of information circumvention procedure and notification to administrator upon detection
- Detection report of web scraping processing
- Detection of antitrust violation and notification to administrator
- Detection of unreported information processing and notification to administrator
- Prevention and blocking of hoarding task assisted by external AI
- Detection of information manipulation on SNS and the like and notification to administrator
- Scoring of user soundness
- Introduction of positive scoring for healthy transactions by BMI-intervened users

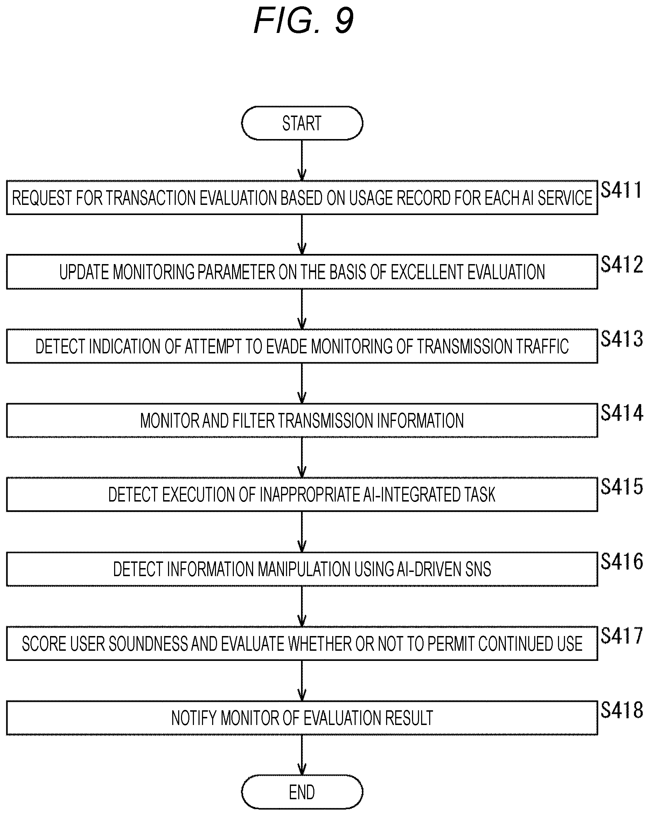

Here, the flow of monitoring processing during the execution of the AI-integrated task that is executed in step S334 in Fig. 8 will be described with reference to the flowchart in Fig. 9. The processing in Fig. 9 can be performed by the monitoring gateway 410 for each user who uses the AI-integrated task.

In step S411, the monitoring gateway 410 requests a transaction evaluation for each AI service on the basis of its usage record.

In step S412, the monitoring gateway 410 updates monitoring parameters used for monitoring transmission information on the basis of excellent evaluations among the requested transaction evaluations.

In step S413, the monitoring gateway 410 detects an indication of a monitoring evasion attempt to evade the monitoring of transmission traffic.

In step S414, the monitoring gateway 410 monitors and filters the transmission information. The monitoring and the filtering may be performed in response to emerging threats such as new forms of misuse and new usage trends that arise over time.

In step S415, the monitoring gateway 410 detects the execution of an inappropriate AI-integrated task among AI-integrated tasks exceeding human abilities.

In step S416, the monitoring gateway 410 detects information manipulation using an AI-driven SNS.

In step S417, the monitoring gateway 410 scores user soundness indicating a degree of soundness of various transactions performed by the user and evaluates whether or not to permit the continued use of the various transactions.

In step S418, the monitoring gateway 410 notifies a monitor monitoring the AI-integrated task through, for example, the monitoring gateway 410 of the evaluation result of evaluating whether or not to permit the continued use of various transactions as monitoring information regarding the AI-integrated task. The evaluation result includes the above-described various flags, the scored user soundness, and the like. However, the purpose here is not to monitor the details of the information content transmitted by an individual. It is necessary to manage information itself exchanged by individuals while ensuring the protection thereof. Therefore, here, it is only required to perform gradual monitoring without directly reading and viewing the content of transmitted information. That is, abnormal or unusual communication activities, fraudulent attempts, and the like, such as use of jargon, access to sites designated for monitoring, an attempt of unauthorized access, transmission of execution codes, and use of transaction sites or services with undisclosed operational status, are subject to intensive monitoring. In a case where an anomaly is observed, the monitor is notified of the abnormality, allowing flexible tracking and confirmation by humans. An anomaly monitoring trap that detects acts of evading monitoring make it easier to detect these acts.

The monitor needs to take action against the user on the basis of the evaluation result notified from the monitoring gateway 410 as needed.

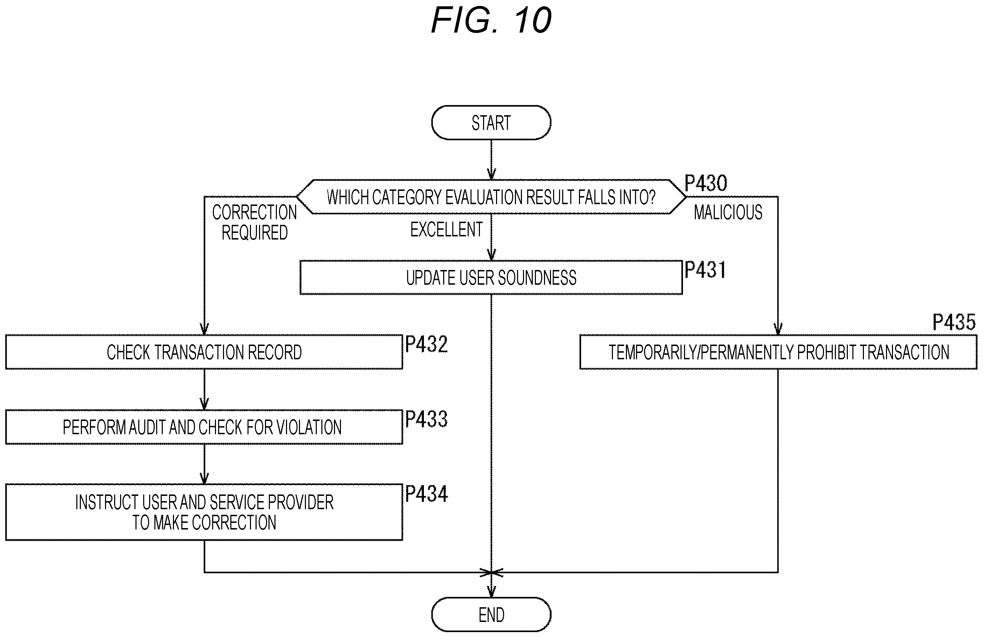

Fig. 10 is a flowchart illustrating an action-taking process performed by the monitor on the basis of the evaluation result.

In process P430, the monitor determines whether the evaluation result notified from the monitoring gateway 410 falls into "excellent", "correction required", or "malicious".

In a case where the evaluation result is "excellent", the processing proceeds to process P431, and the monitor updates the user soundness (user evaluation) of the user scored by the monitoring gateway 410. The updated user soundness is held for each user as an actual value for each AI service.

In a case where the evaluation result is "correction required", the processing proceeds to process P432, and the monitor checks transaction records of the user on the basis of various flags included in the evaluation result.

Next, in process P433, the monitor performs an audit on the transaction records and checks for any violation.

Then, in process P434, the monitor instructs the user and the service provider providing the transaction service where the violation has occurred to correct the violation through notices, warnings, or the like.

In a case where the evaluation result is "malicious", the processing proceeds to process P435, and the monitor temporarily or permanently prohibits transactions between the user and the service provider providing the service used by the user.

Through the above processing, it is possible to prevent the execution of inappropriate tasks by monitoring various transactions by the user who executes the AI-integrated task via the BMI.

### (8-5. Dissemination of information via avatar)

As described above, in the metaverse 450, interaction such as dissemination of information using an avatar may be performed via the BMI. The interaction using the avatar can be achieved through natural language conversations using AI.

By the way, in a general chat function, the intention of the user is electronically converted into text via a keyboard, and the user confirms that the intension has been accurately converted into text information and presses a transmission button, allowing the accurate intention to be transmitted to the other party.

On the other hand, in communication with others, "keeping thoughts that might harm the recipient's impression to oneself and not expressing them verbally" enables smoother social activities. However, direct transmission of information expressing intention via the BMI may cause social problems. The outcome in social life is completely different between wanting to convey one's intention to the other party and actually conveying the intention. Therefore, in a case where a thought result is converted into information that can be transmitted by an external relay device via the BMI, a procedure is required in which the thought result is converted into information recognizable by the user and fed back, and the user confirms that the information reflects what they actually want to convey to the other party, and then transmits the information.

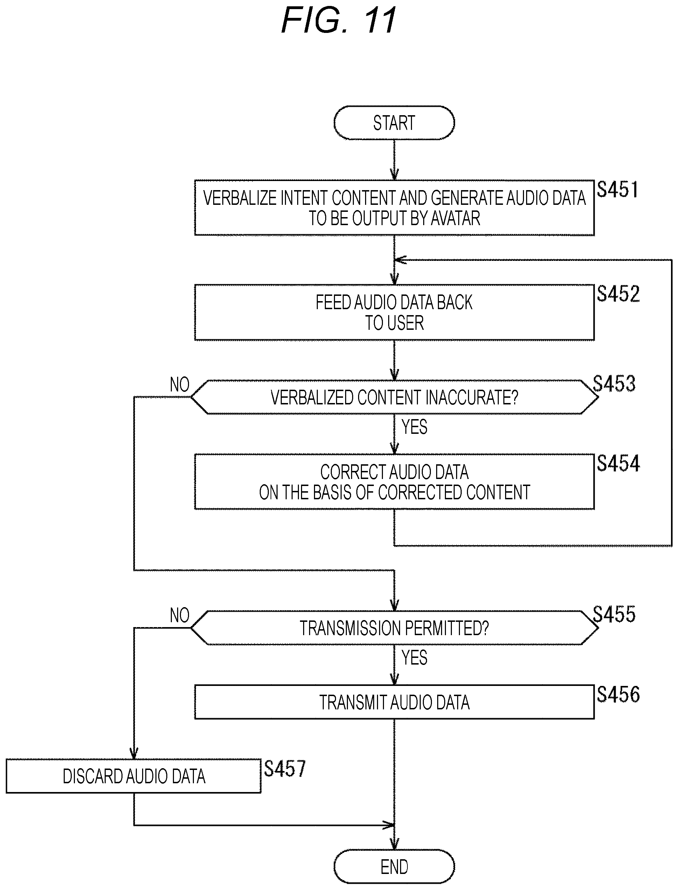

Here, the flow of information dissemination processing via an avatar in the metaverse 450 will be described with reference to Fig. 11. The processing in Fig. 11 is performed by a server on which the metaverse 450 is implemented, and is started when the user transmits his/her intention for information dissemination using the avatar. The server on which the metaverse 450 is implemented also has functions equivalent to those of the gateway device 10 described with reference to Fig. 4.

In step S451, the metaverse 450 verbalizes the intention content of the user represented by the intention signal transmitted via the BMI or the Internet to generate audio data to be output by the avatar on the basis of the verbalized information.

In step S452, the metaverse 450 converts the verbalized information verbalized from the intention of the user into information recognizable by the user, thereby feeding the generated audio data back to the user. The user checks whether the content of the verbalized information verbalized from the intention of the user is inaccurate on the basis of the fed back information. In a case where the content of the verbalized information is inaccurate, an intention signal representing the corrected contents is transmitted to the metaverse 450 via the BMI or the Internet, and in a case where the content of the verbalized information is accurate, an intention signal indicating whether or not to permit the transmission of the audio data (the result of verifying the verbalized information) is transmitted via the BMI or the Internet.

In step S453, the metaverse 450 determines whether or not the verbalized content is inaccurate on the basis of the intention signal transmitted via the BMI or the Internet.

In a case where it is determined in step S453 that the verbalized content is inaccurate, the processing proceeds to step S454, and the metaverse 450 corrects the audio data on the basis of the corrected content represented by the intention signal. Thereafter, the processing returns to step S452, and the subsequent processing is repeated.

On the other hand, in a case where it is determined in step S453 that the verbalized content is accurate, the processing proceeds to step S455, and the metaverse 450 determines whether or not to permit the transmission (whether or not to permit the information dissemination) on the basis of whether or not to permit the transmission of the audio data represented by the intention signal (the result of verifying the verbalized information).

In a case where it is determined in step S455 that the transmission is permitted, the processing proceeds to step S456, and the metaverse 450 transmits the generated audio data to the interaction partner using the avatar.

On the other hand, in a case where it is determined in step S455 that the transmission is denied, the processing proceeds to step S457, and the metaverse 450 discards the verbalized information converted from the intention information together with the generated audio data.

Through the above processing, in a system capable of transmitting intention via the BMI, it is possible to prevent a thought that is not intended to be conveyed to the other party from being detected via the BMI and being conveyed to the other party.

The example of the BMI control in the BMI control system 100 has been described above. Note that any device constituting the BMI control system 100 can be equipped with the functions of the gateway device 10 described with reference to Fig. 4. This enables the prevention of runaway BMI control, which may be caused by the user's consciousness, thoughts, or psychological state.

Note that the configuration and role of the gateway device can be further advanced, and, for example, it may estimate the impact when the information transmitted by the user is conveyed to the recipient and suggest modifications to the information being transmitted to the recipient. Many of the recent information terminals are equipped with a function to present Emojis or character stamps associated with input content as a means of communication. As an advanced use of the function, by providing feedback with characters or the like that represent the recipient's likely impression about the contextually conveyed information, it is possible to avoid causing unintended harm through the careless transmission of information. As described above, by visualizing the impact that may lead to social sanctions and providing feedback to the user to allow the user to recognize and confirm careless transmission of information before the information is shared on an SNS or the like, it is possible to implement a mechanism to prevent the disclosure of such information.

There are temporal and physical steps in the materialization process for actual vocalization or physical text input into a terminal before information is conveyed to the other party. Similarly, in the context of transmitting intention via the BMI, the gateway device is also expected to provide feedback as described above to encourage the user to raise awareness and, in some cases, discourage the user from transmitting his/her intention.

### <9. Hardware configuration example of computer>

The series of processing described above can be performed by hardware or by software. In a case where the series of processing is performed by software, a program included in the software is installed from a program recording medium on a computer incorporated in dedicated hardware, a general-purpose personal computer, or the like.

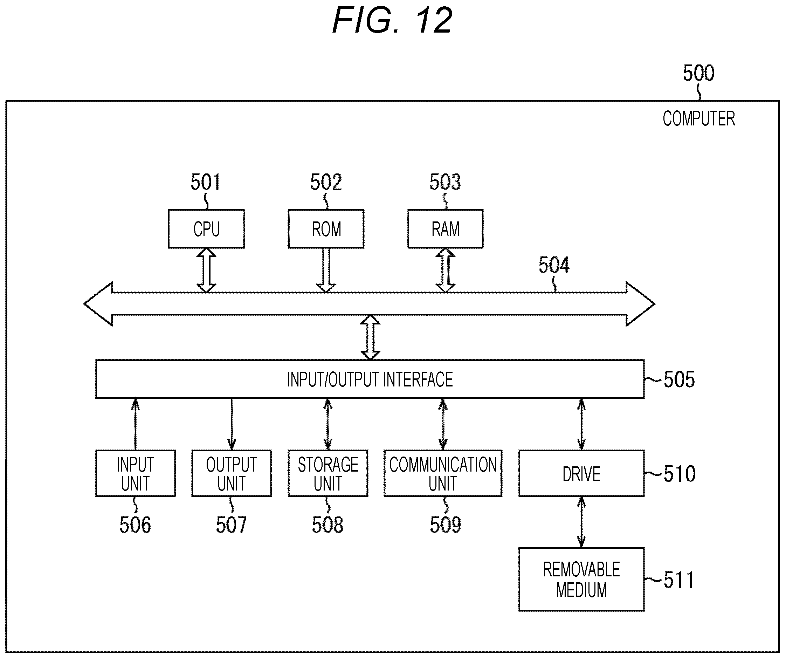

Fig. 12 is a block diagram illustrating a configuration example of hardware of a computer that performs the series of processing described above in accordance with a program. The gateway device 10 and each device constituting the BMI control system 100 and corresponding to the gateway device 10 include, for example, a PC having a configuration similar to the configuration illustrated in Fig. 12.

A central processing unit (CPU) 501, a read only memory (ROM) 502, and a random access memory (RAM) 503 are interconnected via a bus 504.

An input/output interface 505 is further connected to the bus 504. An input unit 506 including a keyboard, a mouse, and the like, and an output unit 507 including a display, a speaker, and the like are connected to the input/output interface 505. Furthermore, a storage unit 508 including a hard disk, a non-volatile memory, or the like, a communication unit 509 including a network interface or the like, and a drive 510 that drives a removable medium 511 are connected to the input/output interface 505.

In the computer configured as described above, for example, the CPU 501 loads the program stored in the storage unit 508 into the RAM 503 via the input/output interface 505 and the bus 504 and execute the program to perform the above-described series of processing.

For example, the program executed by the CPU 501 is recorded in the removable medium 511, or provided via a wired or wireless transmission medium such as a local area network, the Internet, or digital broadcasting, and then installed in the storage unit 508.

The program executed by the computer may be a program in which the processing is performed in time series in the order described in the present specification, or may be a program in which the processing is performed in parallel or at a necessary timing such as when a call is made.

Note that, in the present specification, a system means a set of a plurality of components (devices, modules (parts) and the like), and it does not matter whether or not all the components are in the same housing. Therefore, a plurality of devices housed in separate housings and connected to each other via a network and a single device including a plurality of modules housed in a single housing are both systems.

The effects described in the present specification are merely examples and are not restrictive, and other effects may also be provided.

The embodiment of the present disclosure is not limited to the above-described embodiments, and various modifications can be made without departing from the gist of the present disclosure.

Furthermore, for example, the embodiment of the present disclosure can be configured as cloud computing in which a plurality of devices shares one function and jointly performs processing via a network.

Furthermore, each step described in the flowchart described above can be performed by one device or can be performed by a plurality of devices in a shared manner.

Moreover, in a case where a plurality of pieces of processing is included in one step, the plurality of pieces of processing included in the one step can be performed by one device or performed by a plurality of devices in a shared manner.

The effects described in the present specification are merely examples and are not restrictive, and other effects may be provided.

Moreover, the technology according to the present disclosure can have the following configurations.
(1) A signal processing device including:
   a determination unit that determines whether or not to permit interaction of a user based on a neural signal corresponding to an intention of the user acquired from a brain of the user via a brain machine interface (BMI).
(2) The signal processing device according to (1), in which
   the interaction includes operation of an external device connected in a wired or wireless manner, and
   the determination unit determines whether or not to permit input of a control signal converted from the neural signal to the external device.
(3) The signal processing device according to (2), in which
   the determination unit determines whether or not to permit the input of the control signal to the external device according to presence or absence of a risk due to a malfunction of the external device.
(4) The signal processing device according to (3), in which
   the determination unit determines whether or not to permit the input of the control signal to the external device on the basis of a consciousness state of the user.
(5) The signal processing device according to (3), in which
   the determination unit determines whether or not to permit the input of the control signal to the external device on the basis of an instruction input by a third party.
(6) The signal processing device according to any one of (2) to (5), in which
   the external device includes at least one of a wearable device, a mobile device, a calling device, a display device, an audio output device, an air conditioner, or a mobile entity.
(7) The signal processing device according to (6), in which
   the mobile entity includes at least one of a wheelchair or a vehicle.
(8) The signal processing device according to any one of (1) to (7), further including:
   a generation unit that generates a feedback signal for feeding the intention of the user back to the user on the basis of an intention signal based on the neural signal.
(9) The signal processing device according to (8), in which
   the feedback signal includes at least one of a display signal for display, an audio signal for audio output, or a BMI signal to be input to the brain of the user via the BMI.
(10) The signal processing device according to (1), in which
   the interaction includes use of a network service, and
   the determination unit determines necessity of restricting the use of the network service.
(11) The signal processing device according to (10), in which
   the determination unit determines the necessity of restricting the use of the network service on a basis of a psychological state or thought content of the user.
(12) The signal processing device according to (11), in which
   the determination unit denies or restricts the use of the network service in a case where the user has an abnormal psychological state or may have malicious intent.
(13) The signal processing device according to any one of (10) to (12), in which
   the network service includes at least one of a social networking service (SNS), online shopping, or a financial transaction service.
(14) The signal processing device according to (1), in which
   the interaction includes execution of a task integrated with artificial intelligence (AI) in a network service, and
   the determination unit determines presence of absence of an anomaly in the task to be executed.
(15) The signal processing device according to (14), in which
   the determination unit outputs an alert as monitoring information regarding the network service in a case where the anomaly in the task is detected.
(16) The signal processing device according to (1), in which
   the interaction includes information dissemination using an avatar on a network, and
   the determination unit determines whether or not to permit the information dissemination.
(17) The signal processing device according to (16), in which
   information to be disseminated includes verbalized information verbalized from the intention of the user, and
   the determination unit determines whether or not to permit the information dissemination on the basis of a result of verifying the verbalized information fed back to the user.
(18) The signal processing device according to any one of (1) to (17), in which
   the neural signal includes a signal multiplexed using a probe electrode array arranged in a matrix.
(19) A signal processing method including:
   causing a signal processing device to determine whether or not to permit interaction of a user based on a neural signal corresponding to an intention of the user acquired from a brain of the user via a brain machine interface (BMI).
(20) A signal processing system including:
   a gateway device that determines whether or not to permit interaction of a user based on a neural signal corresponding to an intention of the user acquired from a brain of the user via a brain machine interface (BMI).

### REFERENCE SIGNS LIST

- 10: Gateway device
- 11: Determination unit
- 12: Storage unit
- 100: BMI control system
- 110: Gateway device
- 210: Gateway device
- 310: Wide area network provider device
- 410: Monitoring gateway
- 450: Metaverse

## Claims

1. A signal processing device comprising:
a determination unit that determines whether or not to permit interaction of a user based on a neural signal corresponding to an intention of the user acquired from a brain of the user via a brain machine interface (BMI).

2. The signal processing device according to claim 1, wherein
the interaction includes operation of an external device connected in a wired or wireless manner, and
the determination unit determines whether or not to permit input of a control signal converted from the neural signal to the external device.

3. The signal processing device according to claim 2, wherein
the determination unit determines whether or not to permit the input of the control signal to the external device according to presence or absence of a risk due to a malfunction of the external device.

4. The signal processing device according to claim 3, wherein
the determination unit determines whether or not to permit the input of the control signal to the external device on a basis of a consciousness state of the user.

5. The signal processing device according to claim 3, wherein
the determination unit determines whether or not to permit the input of the control signal to the external device on a basis of an instruction input by a third party.

6. The signal processing device according to claim 2, wherein
the external device includes at least one of a wearable device, a mobile device, a calling device, a display device, an audio output device, an air conditioner, or a mobile entity.

7. The signal processing device according to claim 6, wherein
the mobile entity includes at least one of a wheelchair or a vehicle.

8. The signal processing device according to claim 1, further comprising:
a generation unit that generates a feedback signal for feeding the intention of the user back to the user on a basis of an intention signal based on the neural signal.

9. The signal processing device according to claim 8, wherein
the feedback signal includes at least one of a display signal for display, an audio signal for audio output, or a BMI signal to be input to the brain of the user via the BMI.

10. The signal processing device according to claim 1, wherein
the interaction includes use of a network service, and
the determination unit determines necessity of restricting the use of the network service.

11. The signal processing device according to claim 10, wherein
the determination unit determines the necessity of restricting the use of the network service on a basis of a psychological state or thought content of the user.

12. The signal processing device according to claim 11, wherein
the determination unit denies or restricts the use of the network service in a case where the user has an abnormal psychological state or may have malicious intent.

13. The signal processing device according to claim 10, wherein
the network service includes at least one of a social networking service (SNS), online shopping, or a financial transaction service.

14. The signal processing device according to claim 1, wherein
the interaction includes execution of a task integrated with artificial intelligence (AI) in a network service, and
the determination unit determines presence or absence of an anomaly in the task to be executed.

15. The signal processing device according to claim 14, wherein
the determination unit outputs an alert as monitoring information regarding the network service in a case where the anomaly in the task is detected.

16. The signal processing device according to claim 1, wherein
the interaction includes information dissemination using an avatar on a network, and
the determination unit determines whether or not to permit the information dissemination.

17. The signal processing device according to claim 16, wherein
information to be disseminated includes verbalized information verbalized from the intention of the user, and
the determination unit determines whether or not to permit the information dissemination on a basis of a result of verifying the verbalized information fed back to the user.

18. The signal processing device according to claim 1, wherein
the neural signal includes a signal multiplexed using a probe electrode array arranged in a matrix.

19. A signal processing method comprising:
causing a signal processing device to determine whether or not to permit interaction of a user based on a neural signal corresponding to an intention of the user acquired from a brain of the user via a brain machine interface (BMI).

20. A signal processing system comprising:
a gateway device that determines whether or not to permit interaction of a user based on a neural signal corresponding to an intention of the user acquired from a brain of the user via a brain machine interface (BMI).
